# EUROPEAN PATENT APPLICATION

(11) **EP 1 254 902 A1**
(43) Date of publication of application: **06.11.2002**
(21) Application number: 01900659.2
(22) Date of filing: 11.01.2001
(51) Int. Cl.: C07D 309/32, C07D 493/08, A61K 31/351, A61K 31/357, A61P 43/00, A61P 1/16, A61P 29/00

(54) **AGENTS CORRECTING GENE EXPRESSION REGULATORY ERROR**

(30) Priority: 13.01.2000 JP 2000004989; 03.10.2000 JP 2000303711
(71) Applicant: TAKARA BIO INC., Otsu-shi, Shiga 520-2193 (JP)
(72) Inventor: ENOKI, Tatsuji, Otsu-shi, Shiga 520-0865 (JP); YAMASHITA, Syusaku, Otsu-shi, Shiga 520-2134 (JP); NISHIMURA, Kaori, Otsu-shi, Shiga 520-0853 (JP); SAGAWA, Hiroaki, Kusatsu-shi,Shiga 525-0025 (JP); KATO, Ikunoshin, Uji-shi, Kyoto 611-0028 (JP)
(74) Representative: VOSSIUS & PARTNER
(86) International application number: JP0100082
(87) International publication number: WO01051480

(57) **Abstract**

The present invention provides a suppressor for unbalance of gene expressions, characterized in that the suppressor comprises, as an effective ingredient, a specified compound; a therapeutic agent or prophylactic agent for a disease that requires modulating unbalance of expressions of a specified gene; and a method for modulating unbalance of gene expressions, characterized by administering to a mammal the above-mentioned compound.

SEQUENCE LISTING

<110> Takara Shuzo Co., Ltd.

<120> Therapeutic agents

<130> 01-011-PCT

<150> JP 2000-4989
<151> 2000-01-13

<150> JP 2000-303711
<151> 2000-10-03

<160> 61

<210> 1
<211> 17
<212> DNA
<213> Artificial Sequence

<220>
<223> Designed primer based on nucleotide sequence of human prostaglandi
n G/H synthase-2 mRNA.

<400> 1
acggtgaaac tctggct                                                    17

<210> 2
<211> 17
<212> DNA
<213> Artificial Sequence

<220>
<223> Designed primer based on nucleotide sequence of human prostaglandi
n G/H synthase-2 mRNA.

<400> 2
ggatgctcct gtttaag                                                    17

<210> 3
<211> 20
<212> DNA
<213> Artificial Sequence

<220>
<223> Designed primer based on nucleotide sequence of human macrophage i
nflammatory protein-1-α mRNA.

<400> 3
acattccgtc acctgctcag                                                 20

<210> 4
<211> 20
<212> DNA
<213> Artificial Sequence

<220>
<223> Designed primer based on nucleotide sequence of human macrophage i
nflammatory protein-1-α mRNA.

<400> 4
ggtcgctgac atatttctgg                                                    20

<210> 5
<211> 20
<212> DNA
<213> Artificial Sequence

<220>
<223> Designed primer based on nucleotide sequence of human RANTES mRNA.

<400> 5
ctccacaggt accatgaagg                                                    20

<210> 6

<211> 20

<212> DNA

<213> Artificial Sequence

<220>

<223> Designed primer based on nucleotide sequence of human RANTES mRNA.

<400> 6

gtaagttcag gttcaaggac                                                    20

<210> 7

<211> 20

<212> DNA

<213> Artificial Sequence

<220>

<223> Designed primer based on nucleotide sequence of human β-actin mRN

A.

<400> 7

tggccgtacc actggcatcg                                                    20

<210> 8

<211> 22

<212> DNA

<213> Artificial Sequence

<220>
<223> Designed primer based on nucleotide sequence of human β-actin mRN
A.

<400> 8
tccttctgca tcctgtcggc aa                                                22

<210> 9
<211> 20
<212> DNA
<213> Artificial Sequence

<220>
<223> Designed primer based on nucleotide sequence of human interleukin-
6 mRNA.
<400> 9
ccactcacct cttcagaacg                                                   20

<210> 10
<211> 20
<212> DNA
<213> Artificial Sequence

<220>

<223> Designed primer based on nucleotide sequence of human interleukin-
6 mRNA.
<400> 10
gatgagttgt catgtcctgc                                                    20

<210> 11
<211> 20
<212> DNA
<213> Artificial Sequence

<220>
<223> Designed primer based on nucleotide sequence of human interleukin-
1α mRNA.

<400> 11
gctgcatgga tcaatctgtg                                                    20

<210> 12
<211> 20
<212> DNA
<213> Artificial Sequence

<220>
<223> Designed primer based on nucleotide sequence of human interleukin-
1α mRNA.

<400> 12
tcttcatctt gggcagtcac                                                    20

<210> 13
<211> 20
<212> DNA
<213> Artificial Sequence

<220>
<223> Designed primer based on nucleotide sequence of human interleukin-
1β mRNA.

<400> 13
tggcagaagt acctaagctc                                                    20

<210> 14
<211> 20
<212> DNA
<213> Artificial Sequence

<220>
<223> Designed primer based on nucleotide sequence of human interleukin-
1β mRNA.

<400> 14
catatggacc agacatcacc                                                    20

<210> 15
<211> 20
<212> DNA
<213> Artificial Sequence

<220>
<223> Designed primer based on nucleotide sequence of human tumor necros
is factor α mRNA.

<400> 15
aaaggacacc atgagcactg                                                    20

<210> 16
<211> 20
<212> DNA
<213> Artificial Sequence

<220>
<223> Designed primer based on nucleotide sequence of human tumor necros
is factor α mRNA.

<400> 16
cgagaagatg atctgactgc                                                    20

<210> 17

<211> 20

<212> DNA

<213> Artificial Sequence

<220>

<223> Designed primer based on nucleotide sequence of human interleukin-

7 receptor mRNA.

<400> 17

tggagaaagt ggctatgctc                                                    20

<210> 18

<211> 20

<212> DNA

<213> Artificial Sequence

<220>

<223> Designed primer based on nucleotide sequence of human interleukin-

7 receptor mRNA.

<400> 18

ggcggtaagc tacatcatgc                                                    20

<210> 19

<211> 20

<212> DNA

<213> Artificial Sequence

<220>
<223> Designed primer based on nucleotide sequence of human macrophage i
nflammatory protein-1-β mRNA.

<400> 19
gaagcttctg agttctgcag                                                    20

<210> 20
<211> 20
<212> DNA
<213> Artificial Sequence

<220>
<223> Designed primer based on nucleotide sequence of human macrophage i
nflammatory protein-1-β mRNA.

<400> 20
ttcctgtctc tgagcagctc                                                    20

<210> 21
<211> 20
<212> DNA
<213> Artificial Sequence

<220>
<223> Designed primer based on nucleotide sequence of human liver and ac
tivation-regulated chemokine mRNA.

<400> 21
aagagtttgc tcctggctgc                                                    20

<210> 22
<211> 20
<212> DNA
<213> Artificial Sequence

<220>
<223> Designed primer based on nucleotide sequence of human liver and ac
tivation-regulated chemokine mRNA.

<400> 22
ttggatttgc gcacacagac                                                    20

<210> 23
<211> 20
<212> DNA
<213> Artificial Sequence

<220>
<223> Designed primer based on nucleotide sequence of human macrophage-d

erived chemokine mRNA.

<400> 23
acagactgca ctcctggttg                                                    20

<210> 24
<211> 20
<212> DNA
<213> Artificial Sequence

<220>
<223> Designed primer based on nucleotide sequence of human macrophage-d
erived chemokine mRNA.

<400> 24
aggctcttca ttggctcagc                                                    20

<210> 25
<211> 19
<212> DNA
<213> Artificial Sequence

<220>
<223> Designed primer based on nucleotide sequence of human macrophage i
nflammatory protein-2-β mRNA.

<400> 25
cgtggtcact gaactgcgc                                                    19

<210> 26
<211> 20
<212> DNA
<213> Artificial Sequence

<220>
<223> Designed primer based on nucleotide sequence of human macrophage i
nflammatory protein-2-β mRNA.

<400> 26
acttctctcc tgtcagttgg                                                   20

<210> 27
<211> 18
<212> DNA
<213> Artificial Sequence

<220>
<223> Designed primer based on nucleotide sequence of human macrophage i
nflammatory protein-2-α mRNA.

<400> 27
tgctgctcct gctcctgg                                                     18

<210> 28

<211> 20

<212> DNA

<213> Artificial Sequence

<220>

<223> Designed primer based on nucleotide sequence of human macrophage i

nflammatory protein-2-α mRNA.

<400> 28

ctgcccattc ttgagtgtgg                                                    20

<210> 29

<211> 21

<212> DNA

<213> Artificial Sequence

<220>

<223> Designed primer based on nucleotide sequence of human growth regul

ated protein-1 mRNA.

<400> 29

aacatccaaa gtgtgaacgt g                                                  21

<210> 30

<211> 19

<212> DNA

<213> Artificial Sequence

<220>

<223> Designed primer based on nucleotide sequence of human growth regul

ated protein-1 mRNA.

<400> 30

ctctgcagct gtgtctctc                                                    19

<210> 31

<211> 20

<212> DNA

<213> Artificial Sequence

<220>

<223> Designed primer based on nucleotide sequence of human matrix metal

loproteinase-9 mRNA.

<400> 31

cttctccaga agcaactgtc                                                   20

<210> 32

<211> 20

<212> DNA

<213> Artificial Sequence

<220>
<223> Designed primer based on nucleotide sequence of human matrix metal
loproteinase-9 mRNA.

<400> 32
accacaactc gtcatcgtcg                                                    20

<210> 33
<211> 20
<212> DNA
<213> Artificial Sequence

<220>
<223> Designed primer based on nucleotide sequence of human migration in
hibitory factor-related protein-8 mRNA.

<400> 33
tcatcgacgt ctaccacaag                                                    20

<210> 34
<211> 20
<212> DNA
<213> Artificial Sequence

<220>
<223> Designed primer based on nucleotide sequence of human migration in
hibitory factor-related protein-8 mRNA.

<400> 34
accagaatga ggaactcctg                                                    20

<210> 35
<211> 20
<212> DNA
<213> Artificial Sequence

<220>
<223> Designed primer based on nucleotide sequence of human lyzozyme mRN
A.

<400> 35
tgtcctcctt tctgttacgg                                                    20

<210> 36
<211> 20
<212> DNA
<213> Artificial Sequence

<220>
<223> Designed primer based on nucleotide sequence of human lyzozyme mRN

A.

<400> 36
tgacaggcat taactgctcc                                                    20

<210> 37
<211> 20
<212> DNA
<213> Artificial Sequence

<220>
<223> Designed primer based on nucleotide sequence of human GABA(A) rece
ptor-associated protein mRNA.

<400> 37
aagaagagca tccgttcgag                                                    20

<210> 38
<211> 20
<212> DNA
<213> Artificial Sequence

<220>
<223> Designed primer based on nucleotide sequence of human GABA(A) rece
ptor-associated protein mRNA.

<400> 38
tggtgttcct ggtacagctg                                                    20

<210> 39
<211> 19
<212> DNA
<213> Artificial Sequence

<220>
<223> Designed primer based on nucleotide sequence of human interferon-i
nduced 17-kDa/15-kDa protein mRNA.

<400> 39
catgtcggtg tcagagctg                                                     19

<210> 40
<211> 20
<212> DNA
<213> Artificial Sequence

<220>
<223> Designed primer based on nucleotide sequence of human interferon-i
nduced 17-kDa/15-kDa protein mRNA.

<400> 40
ctcacttgct gcttcaggtg                                                    20

<210> 41

<211> 20

<212> DNA

<213> Artificial Sequence

<220>

<223> Designed primer based on nucleotide sequence of human interferon-i

nducible protein p78 mRNA.

<400> 41

gtggctgaga acaacctgtg                                                    20

<210> 42

<211> 20

<212> DNA

<213> Artificial Sequence

<220>

<223> Designed primer based on nucleotide sequence of human interferon-i

nducible protein p78 mRNA.

<400> 42

agtcagatcc gggacatctc                                                    20

<210> 43

<211> 20
<212> DNA
<213> Artificial Sequence

<220>
<223> Designed primer based on nucleotide sequence of human SCO(cytochro
me oxidase deficient, yeast) homolog-2 mRNA.

<400> 43
gcagcagcaa aagcgaacag                                                    20

<210> 44
<211> 20
<212> DNA
<213> Artificial Sequence

<220>
<223> Designed primer based on nucleotide sequence of human SCO(cytochro
me oxidase deficient, yeast) homolog-2 mRNA.

<400> 44
gatgaagaca ggctgcactg                                                    20

<210> 45
<211> 20
<212> DNA

<213> Artificial Sequence

<220>
<223> Designed primer based on nucleotide sequence of human transketolas
e mRNA.
<400> 45
cctacgtatc agctccatcc                                                    20

<210> 46
<211> 20
<212> DNA
<213> Artificial Sequence

<220>
<223> Designed primer based on nucleotide sequence of human transketolas
e mRNA.
<400> 46
catacagagc cctctgacag                                                    20

<210> 47
<211> 20
<212> DNA
<213> Artificial Sequence

<220>
<223> Designed primer based on nucleotide sequence of human adenosine A2

a receptor mRNA.

<400> 47
tgtggctcaa cagcaacctg                                                    20

<210> 48
<211> 20
<212> DNA
<213> Artificial Sequence

<220>
<223> Designed primer based on nucleotide sequence of human adenosine A2
a receptor mRNA.

<400> 48
gaccacatcc tcaaagagac                                                    20

<210> 49
<211> 20
<212> DNA
<213> Artificial Sequence

<220>
<223> Designed primer based on nucleotide sequence of human CD37 antigen
 mRNA.

<400> 49
gcctcatcaa gtacttcctc                                                    20

<210> 50
<211> 20
<212> DNA
<213> Artificial Sequence

<220>
<223> Designed primer based on nucleotide sequence of human CD37 antigen
 mRNA.

<400> 50
atgaggactt ggaaccagtc                                                    20

<210> 51
<211> 20
<212> DNA
<213> Artificial Sequence

<220>
<223> Designed primer based on nucleotide sequence of human properdin P
factor, complement mRNA.

<400> 51
ctaccagaaa cgtagtggtg                                                    20

<210> 52
<211> 20
<212> DNA
<213> Artificial Sequence

<220>
<223> Designed primer based on nucleotide sequence of human properdin P
factor, complement mRNA.

<400> 52
gtgtctcctt aggttcgtgg                                                    20

<210> 53
<211> 20
<212> DNA
<213> Artificial Sequence

<220>
<223> Designed primer based on nucleotide sequence of human regulator of
 G-protein signaling-2 mRNA.

<400> 53
ttggctgttc aacacgactg                                                    20

<210> 54

<211> 20

<212> DNA

<213> Artificial Sequence

<220>

<223> Designed primer based on nucleotide sequence of human regulator of
 G-protein signaling-2 mRNA.

<400> 54

ggcagttgta aagcagccac                                                    20

<210> 55

<211> 20

<212> DNA

<213> Artificial Sequence

<220>

<223> Designed primer based on nucleotide sequence of human Nef-associat
ed factor-1 mRNA.

<400> 55

agcagaattc accagagagc                                                    20

<210> 56

<211> 20

<212> DNA

<213> Artificial Sequence

<220>
<223> Designed primer based on nucleotide sequence of human Nef-associat
ed factor-1 mRNA.

<400> 56
ttccatcttc ggtgagcctg                                                    20

<210> 57
<211> 20
<212> DNA
<213> Artificial Sequence

<220>
<223> Designed primer based on nucleotide sequence of human myeloid leuk
emia cell differentiation protein-1 mRNA.

<400> 57
gcatgcttcg gaaactggac                                                    20

<210> 58
<211> 20
<212> DNA
<213> Artificial Sequence

<220>

<223> Designed primer based on nucleotide sequence of human myeloid leuk

emia cell differentiation protein-1 mRNA.

<400> 58

gaagttacag cttggagtcc                                                    20

<210> 59

<211> 20

<212> DNA

<213> Artificial Sequence

<220>

<223> Designed primer based on nucleotide sequence of human signal pepti

dase complex (18kDa) mRNA.

<400> 59

gattgtagtg gtgctcagtg                                                    20

<210> 60

<211> 20

<212> DNA

<213> Artificial Sequence

<220>

<223> Designed primer based on nucleotide sequence of human signal pepti

dase complex (18kDa) mRNA.

<400> 60
tggcatcttc ccaggaacag                                                    20

<210> 61
<211> 21
<212> DNA
<213> Artificial Sequence

<220>
<223> Designed primer based on nucleotide sequence of human β-actin mRN
A.

<400> 61
caagagatgg ccacggctgc t                                                  21

## Description

### TECHNICAL FIELD

The present invention relates to utilization of a physiologically active substance derived from natural product, and to a medicament comprising the physiologically active substance as an effective ingredient.

### PRIOR ART

Interleukins, a class of polypeptide substances that mediate intercellular interactions associated with immune reactions, are known to occur as interleukins 1 to 18, each of which contributes to sustaining homeostasis of a living body. The interleukins refer to a group of molecular species of cytokines that mediate, control action for inflammatory reaction or immunity, antitumor action, or the like.

As to interleukin 6 (IL-6) among the interleukins, its cDNA has been cloned as a differentiation factor for inducing the final differentiation process of B cell. More recently, it has been elucidated that IL-6 is involved not only in immune responses but also in the cell differentiation and acute reactions in the hematopoietic system and the nervous system. Further, it has been clarified that IL-6 also has a close association with the onset of various immune disorders, inflammatory diseases and lymphatic tumors.

IL-6 induces B cell to produce antibody, so that immunoglobulin of each of classes of IgM, IgG and IgA is produced. However, unlike interleukin-4, IL-6 is not involved in class switches. IL-6 also functions as a growth factor for B cell and plasmacyte. Additionally, IL-6 is involved in T cell system to proliferate or differentiate T cell. Furthermore, IL-6 is involved in the hematopoietic system, and IL-6 shortens the G0 phase in cooperation with interleukin-3, thereby proliferating hematopoietic stem cell. IL-6 also promotes megakaryocyte maturation to induce an increase in platelet count.

Furthermore, IL-6 is involved in acute reactions in which a living body responds immediately to a bacterial or viral infection, a malignant tumor, or the like. IL-6 is also involved in the nervous system, in which IL-6 is secreted from nervous system cell such as glioblastoma and astrocytoma, and functions in the induction of differentiation of the nervous system [Term Library "Cytokines and Growth Factors", extra issue of "Jikken Igaku" (Experimental Medicine), 28-29, (1995), Yodosha Co., Ltd.].

In addition, interleukin-10 (IL-10) has been known as a type of cytokine that inhibits the synthesis of a broad range of cytokines. IL-10 is produced by type-II helper cell and indirectly inhibits Th1 cell-produced cytokines by inhibiting the antigen-presenting cell. Therefore, IL-10 production in excess would inhibit the interferon γ production or the like, thereby leading to an immunocompromised state.

Besides them, chemokines, a group of factors showing chemotaxis for leukocyte, have been shown to have a close association with allergic inflammation and autoimmune diseases. Chemokines are roughly classified into two families according to the position of cysteine in their amino acid sequences: CXC chemokines and CC chemokines. The former includes, for instance, macrophage inflammatory protein-2-β (MIP2β, macrophage inflammatory protein-2-α (MIP2α), growth-regulated protein-1 (Gro1), and the like, and the latter includes, for instance, macrophage inflammatory protein-1-α (MIP1α), RANTES (regulated on activation, normal T cell expressed and secreted), macrophage inflammatory protein-1-β (MIP1β), liver and activation-regulated chemokine (LARC), macrophage-derived chemokine (MDC), and the like.

Also, tumor necrosis factor α (TNFα) has been known to directly cause inflammation in autoimmune diseases such as rheumatoid arthritis and in inflammatory diseases.

As described above, cytokines are important for sustaining homeostasis of a living body. However, when its production is in excess, cytokines can cause various diseases involving immunity or inflammatory reactions.

In addition, there has been known that arachidonic acid metabolism is largely involved in the causation of inflammation and ache in living tissue, together with these cytokine actions. Therefore, it is considered that the modulation of the actions of substances involved in the metabolism, for instance, prostaglandin G/H synthetase-2 (COX2) or the like, is also important in its association with the above-mentioned diseases.

On the other hand, active oxygen is also largely associated with inflammatory diseases and the like. Active oxygens can be roughly classified radical and non-radical active oxygens. The radical active oxygen includes hydroxy radical, hydroxyperoxy radical, peroxy radical, an alkoxy radical, nitrogen dioxide, nitrogen monoxide, thyl radical, and superoxide. On the other hand, the non-radical active oxygen includes singlet oxygen, hydrogen peroxide, lipid hydroperoxide, hypochlorous acid, ozone, and peroxynitrous acid. All these active oxygens are associated with a large number of pathologic conditions, including various inflammatory diseases, diabetes mellitus, cancers, arteriosclerosis, nerve diseases, and ischemic reperfusion disorders.

Active oxygen is constantly produced at a low concentration via a number of pathways in a living body. Any of these active oxygens, such as superoxide and hydrogen peroxide that are physiologically lost from the electron transmission system of mitochondria and the like; hydroxy radicals produced by the catalytic action of a transition metal such as copper or iron; hypochlorous acid produced by neutrophils, monocytes, and the like for protection against infection, and NO produced upon arginine degradation, are unavoidably produced. Since the living body has various enzymes and low-molecular compounds functioning as the active oxygen elimination system against these active oxygens produced, the living body keeps a good balance between their production and elimination. However, if the active oxygen production system becomes dominant over the active oxygen elimination system as a result of the activation of the production system or conversely as a result of the inactivation of the elimination system under certain causes, the living body would undergo oxidative disorder. Such a condition is referred to as "oxidation stress." In addition to such an unbalance within a living body, the living body is always exposed to oxidation stress from sources outside the living body, such as atmosphere and food, so that the oxidation stress is unavoidable in daily life.

Hemeoxygenase (HO) is an *in vivo* enzyme involved in bilirubin production. As HO, there have been known to exist two isozymes: hemeoxygenase-1 (HO1) and hemeoxygenase-2 (HO2). The above-mentioned bilirubin also possesses activities as an antioxidative substance, including antioxidative action of a fatty acid, scavenging action of a lipid radical, inhibitory action of production of hydroperoxide of phospholipids, neutral fats and cholesterol by oxygen radicals generated in a large amount as a result of neutrophil phagocytosis or the like, inhibitory action of LDL (low-density lipoprotein) production which is closely associated with onset of arteriosclerosis, scavenging action of singlet oxygen, and the like. Therefore, the bilirubin plays a key role as an endogenous antioxidative substance in a living body. Various radicals act on a variety of biological substances such as proteins and nucleic acids as well as lipids to cause chronic diseases and cancers, but various radicals mentioned above are diminished by bilirubin [Bioscience of Porphyrin-Heme-Prospects for Application to Genetic Disease and Cancer Engineering -, Gendai Igaku, extra issue 27 (1995), Tokyo Kagaku Dojin Co., Ltd.]. In other words, a reduction in HO activity or a reduction in expression of the HO gene hampers adaptation of the living body to oxidation stress. It is considered, therefore, that the induction of HO production can contribute to the antioxidative action and anti-inflammatory action in the living body.

In addition, the above-mentioned RANTES, a kind of cytokine, has been reported to enhance the production of active oxygen by eosinophil, and similar effects to those of the induction of HO production can be expected by inhibition of RANTES production.

In a stressed state or in a transition state to a disease, the homeostasis-sustaining function of a living body is found to be disordered. Various functions and various biological factors as mentioned above are compoundly involved in the sustenance of homeostasis in a living body. Therefore, malignant changes in the gene expression conditions (unbalance of gene expressions) are basically present in the state. However, the details of the modulation of gene expressions at the gene level, for instance, those in a stressed state or during its avoidance have not yet been elucidated. Therefore, there have not been found any means of avoiding the unbalance of gene expressions by the modulation at the gene level.

### DISCLOSURE OF INVENTION

In the sustenance of homeostasis in a living body, interleukins and *in vivo* enzymes that act to eliminate active oxygen have been involved, and a variety of diseases are caused by disorders in the production of individual interleukins or *in vivo* enzymes.

An object of the present invention is to provide a pharmaceutical composition that regulates the production of various biological factors such as interleukins and *in vivo* enzymes, so that the pharmaceutical composition is useful in the sustenance and recovery of homeostasis in a living body.

Summarizing the present invention, a first invention of the present invention pertains to a suppressor for unbalance of gene expressions, characterized in that the suppressor comprises, as an effective ingredient, at least one compound selected from a compound represented by the following formula (I): 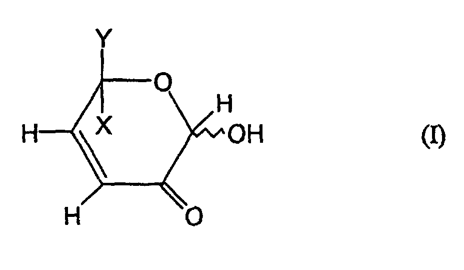 wherein X and Y are H or CH₂OH, with proviso that when X is CH₂OH, Y is H, and when X is H, Y is CH₂OH;
a compound represented by the following formula (II): 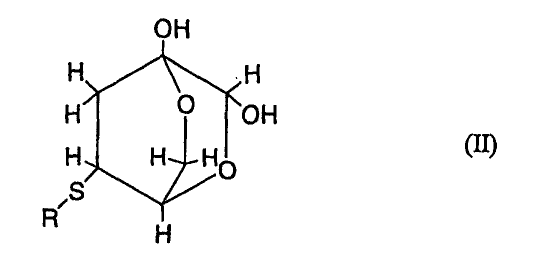 wherein R is a residue resulting from removal of one SH group from an SH-group containing compound;
and a salt thereof. In the first invention of the present invention, as the gene which can be modulated for the unbalance of expressions by the use of the suppressor for unbalance of gene expressions, there are exemplified, for instance, by the following 31 kinds of a group of genes, concretely, one or more genes selected from the group consisting of:
interleukin-6 (GenBank Accession NO: M14584) gene,
interleukin-10 (GenBank Accession NO: M57627) gene,
hemeoxygenase-1 (GenBank Accession NO: NM 002133) gene,
prostaglandin G/H synthase-2 (GenBank Accession NO: AL033533) gene,
macrophage inflammatory protein-1-α (GenBank Accession NO: M23452) gene,
RANTES (GenBank Accession NO: NM 002985) gene,
interleukin-1α (GenBank Accession NO: X02851) gene,
interleukin-1β (GenBank Accession NO: K02770) gene,
tumor necrosis factor α (GenBank Accession NO: M10988) gene,
interleukin-7 receptor (GenBank Accession NO: M29696) gene,
macrophage inflammatory protein-1-β (GenBank Accession NO: J04130) gene,
liver and activation-regulated chemokine (GenBank Accession NO: D86955) gene,
macrophage-derived chemokine (GenBank Accession NO: U83171) gene,
macrophage inflammatory protein-2-β (GenBank Accession NO: M36821) gene,
macrophage inflammatory protein-2-α (GenBank Accession NO: M36820) gene,
growth regulated protein-1 (GenBank Accession NO: J03561) gene,
matrix metalloproteinase-9 (GenBank Accession NO: J05070) gene,
migration inhibitory factor-related protein-8 (GenBank Accession NO: X06234) gene,
lyzozyme (GenBank Accession NO: M21119) gene,
GABA(A) receptor-associated protein (GenBank Accession NO: NM 007278) gene,
interferon-induced 17-kDa/15-kDa protein (GenBank Accession NO: M13755) gene,
interferon-inducible protein p78 (GenBank Accession NO: M33882) gene, SCO(cytochrome oxidase deficient, yeast) homolog-2 (GenBank Accession NO: AL021683) gene,
transketolase (GenBank Accession NO: L12711) gene,
adenosine A2a receptor (GenBank Accession NO: S46950) gene,
CD37 antigen (GenBank Accession NO: X14046) gene,
properdin P factor, complement (GenBank Accession NO: M83652) gene,
regulator of G-protein signaling-2 (GenBank Accession NO: L13463) gene,
Nef-associated factor-1 (GenBank Accession NO: AJ011896) gene,
myeloid leukemia cell differentiation protein-1 (GenBank Accession NO: L08246) gene, and
signal peptidase complex (18 kDa) (GenBank Accession NO: AF061737) gene.

A second invention of the present invention pertains to a therapeutic agent or prophylactic agent for a disease that requires modulating unbalance of expressions of one or more genes selected from the above-mentioned 31 kinds of a group of genes, characterized in that the therapeutic agent or prophylactic agent comprises as an effective ingredient a compound selected from the compound represented by the formula (I), the compound represented by the formula (II), and a salt thereof. The therapeutic agent or prophylactic agent is especially preferable when the disease that requires modulating unbalance of expressions of the above-mentioned genes is an inflammatory disease. In addition, one embodiment of the therapeutic agent or prophylactic agent includes, for instance, an inhibitor for interleukin-6 production, an inhibitor for interleukin-10 production, an inducer for hemeoxygenase production, an inhibitor for prostaglandin G/H synthase-2 production, an inhibitor for macrophage inflammatory protein-1-α production, an inhibitor for RANTES production, an inhibitor for tumor necrosis factor α production, and the like, which can be especially suitably used as an inhibitor or inducer for production of various biological factors concretely exemplified herein.

The term "induce" as used herein encompasses the meaning of "enhance" which refers to an increase in the amount of a desired substance in a living body as compared to that before the administration of the medicament.

A third invention of the present invention pertains to a method for modulating unbalance of gene expressions, characterized by administering to a mammal at least one compound selected from the compound represented by the formula (I), the compound represented by the formula (II), and a salt thereof. The genes which can be modulated for the unbalance of gene expressions according to the third invention of the present invention are exemplified, for instance, by one or more genes selected from the above-mentioned 31 kinds of a group of genes.

### BRIEF DESCRIPTION OF THE DRAWINGS

Figure 1 is a graph showing a concentration of IL-6 in the culture supernatant when the cell is cultured under each of the culture conditions.
Figure 2 is a graph showing a concentration of IL-10 in the culture supernatant when the cell is cultured under each of the culture conditions.
Figure 3 is an electrophoretic diagram on agarose gel showing the results of amplification of COX2 mRNA-derived cDNA by PCR.
Figure 4 is an electrophoretic diagram on agarose gel showing the results of amplification of MIP1α mRNA-derived cDNA by PCR.
Figure 5 is an electrophoretic diagram on agarose gel showing the results of amplification of RANTES mRNA-derived cDNA by PCR.
Figure 6 is a graph showing a concentration of TNFα in the culture supernatant when the cell is cultured under each of the conditions of with or without addition of DGE.
Figure 7 is a graph showing a concentration of TNFα in the culture supernatant when the cell is cultured under each of the conditions of with or without addition of agarobiose.

### BEST MODE FOR CARRYING OUT THE INVENTION

In the present invention, there is used as an effective ingredient at least one compound selected from the compound represent by the above-mentioned formula (I), the compound represent by the above-mentioned formula (II), and salts thereof. Also, the effective ingredient may be a derivative of the compound that is capable of functioning as a prodrug as described below. Therefore, the effective ingredients according to the present invention encompass the derivatives of the compound of the above-mentioned formula (I) and (II) and salts thereof, as long as the desired effects of the present invention can be obtained.

The compound represent by the formula (I) used in the present invention can be obtained by keeping a compound having 3,6-anhydrogalactose at a reducing end thereof, such as agarobiose or κ-carrabiose, under neutral to alkaline conditions. The compound can also be prepared by subjecting a compound containing 3,6-anhydrogalactose in its structure to acid hydrolysis at a pH of less than 7 and/or enzymolysis, and subsequently keeping the resulting acid hydrolyzate and/or enzymolyzate under neutral to alkaline conditions. The compound containing 3,6-anhydrogalactose in its structure includes, for instance, agarobiose, agarotetraose, agarohexaose, agarooctaose, κ-carrabiose and the like.

Upon keeping the compound containing 3,6-anhydrogalactose at a reducing end thereof, such as agarobiose or κ-carrabiose, under neutral to alkaline conditions at a pH of 7 or higher, the composition of the reaction mixture in which at least one compound selected from these compounds is dissolved or suspended is not particularly limited. There can be preferably used a solution prepared by dissolving an alkali exemplified by, but is not particularly limited to, an inorganic base such as sodium hydroxide, potassium hydroxide, calcium hydroxide or ammonia, or an organic base such as Tris, ethylamine or triethylamine, in water (for instance, distilled water, ion-exchanged water, tap water) as a solvent. The concentration of the alkali is not particularly limited. The alkali can be used in a concentration of preferably from 0.0001 to 5 N, more preferably from 0.001 to 1 N. In addition, the reaction temperature is preferably, but is not particularly limited to, from 0° to 200°C, more preferably from 20° to 130°C. The reaction time is preferably, but is not particularly limited to, several seconds to several days. The kinds and concentration of the alkali, the reaction temperature and the reaction time, and the amount of the above-mentioned compound to be dissolved or suspended as a raw material in the reaction mixture may be properly chosen depending upon the kinds of the compound and the amount of the desired compound of the formula (I) formed. In general, the pH is 7 or higher. The formation of the compound represented by the formula (I) proceeds quickly at a higher alkali concentration than those at a lower alkali concentration, and at a higher temperature than those at a lower temperature.

For instance, the compound represented by the formula (I) is formed by preparing a solution of agarobiose or κ-carrabiose at a pH of 11.5, and keeping the solution at 37°C for 5 minutes.

The resulting alkali solution containing the compound represented by the formula (I) may be used after neutralization according to its purpose, or its pH may be adjusted to a pH of lower than 7 to use the solution as an acidic solution.

When the compound represented by the formula (I) is prepared by subjecting a compound containing 3,6-anhydrogalactose in its structure to acid hydrolysis at a pH of lower than 7 and/or enzymolysis, and thereafter keeping the resulting acid hydrolyzate and/or enzymolyzate under neutral to alkaline conditions in the same manner as described above, the acid hydrolysis may be carried out by preparing a reaction solution, for instance, wherein an acid such as an inorganic acid such as hydrochloric acid, sulfuric acid or nitric acid or an organic acid such as citric acid, formic acid, acetic acid, lactic acid or ascorbic acid is dissolved in water as the solvent so as to have a concentration of the acid of preferably from 0.001 to 5 N, dissolving or suspending a proper amount of the raw material compound in the reaction solution, and carrying out the reaction at a reaction temperature of preferably from 0° to 200°C for a reaction time of preferably from several seconds to several days. In addition, in the case where the enzymolysis is carried out, the reaction may be carried out using the similar reaction solution and reaction conditions to those for the acid hydrolysis, for instance, with a proper amount of the enzyme α-agarase, such as α-agarase described in WO 00/50578, under conditions at which the enzyme exhibits its activity.

As the purification means for the compound represented by the formula (I) of the present invention contained in the reaction solution, there may be employed a known means of purification such as a chemical method or a physical method. These means may be used in combination with such purification methods as gel filtration, fractionation using a molecular weight fractionation membrane, solvent extraction, and various chromatographies using ion exchange resin or the like.

For instance, a compound represented by the formula (I) in which X is CH₂OH and Y is H can be purified from the product obtained by treating agarobiose under neutral to alkaline conditions, and a compound represented by the formula (I) in which X is H and Y is CH₂OH can be purified from the product obtained by treating κ-carrabiose under neutral to alkaline conditions.

The compound represented by the formula (II) used in the present invention can be formed in the reaction solution by reacting the compound represented by the formula (I) with an SH group-containing compound.

The SH group-containing compound used is not particularly limited in any way, as long as the compound has at least one SH group. R in the formula (II) refers to the remaining residue excluding the one SH group consumed in the binding of the SH group-containing compound to the compound represented by the formula (I) by the reaction of the SH group-containing compound with the compound represented by the formula (I). Therefore, when the SH group-containing compound has two or more SH groups, one or more SH groups exist in the residue represented by R. Examples of the SH group-containing compounds include methanethiol, butanethiol, mercaptoethanol, SH group-containing amino acids, SH group-containing amino acid derivatives and the like.

Examples of the SH group-containing amino acids include cysteine and homocysteine. In addition, as the SH group-containing amino acid derivatives, there are exemplified derivatives of the above-mentioned amino acids, such as cysteine derivatives, cysteine-containing peptides and cysteine derivative-containing peptides. The cysteine derivative includes, for instance, amide compounds, acetyl compounds, ester compounds, and the like of cysteine. The cysteine-containing peptide is not particularly limited, as long as cysteine is a constituent in the peptide. The cysteine-containing peptides encompass from oligopeptides, such as low-molecular compounds such as glutathione, to high molecular compounds comprising polypeptides, like proteins. In addition, a peptide containing cystine or homocystine can also be used as a cysteine- or homocysteine-containing peptide in the present invention, under the conditions in which the peptide containing cystine or homocystine is converted to a cysteine- or homocysteine-containing peptide, for instance, the combination with a reducing treatment. The cysteine derivative-containing peptide includes substances in which cysteine of the above-mentioned cysteine-containing peptides is substituted with a cysteine derivative.

The cysteine-containing peptides also encompass cysteine-containing peptides containing a saccharide, a lipid, or the like. The cysteine-containing peptides may be salts, acid anhydrides, esters and the like of various compounds mentioned above.

The reaction of the compound represented by the formula (I) with the SH group-containing compound during the preparation of the compound represented by the formula (II) may be carried out under any known reaction conditions without particular limitation, as long as the SH group of the SH group-containing compound is reactive under the conditions. For instance, the reaction may be carried out by allowing the compound represented by the formula (I) and the SH group-containing compound, such as cysteine or glutathione, to stand in water or a solution such as PBS at a temperature of preferably from 0° to 100°C, more preferably from 30° to 50°C, for preferably from 1 hour to 1 week, more preferably overnight.

As the purification and isolation means for the compound represented by the formula (II) prepared by reacting the compound represented by the formula (I) with the above-mentioned SH group-containing compound, a known purification method such as a chemical method or a physical method can be employed. For instance, various means exemplified as the purification means for the compound represented by the above-mentioned formula (I) may be used in combined for use.

In addition, the compound represented by the formula (I) can also be metabolically converted to the compound represented by the formula (II) used in the present invention, for instance, by the reaction with an SH group-containing compound such as cysteine or glutathione in a living body.

The salt of the compound represented by the above-mentioned formula (I) or (II) is preferably a pharmaceutically acceptable salt and can be converted from the compound by a known method. The salts include, for instance, salts of inorganic acids such as hydrochloric acid, hydrobromic acid, hydroiodic acid and sulfuric acid, salts of organic acids such as formic acid, acetic acid, oxalic acid, malonic acid and succinic acid, and ammonium salts obtained by a reaction with an alkyl halide such as methyl iodide or a benzyl halide.

Various isomers such as optical isomers, keto-enol tautomers and geometrical isomers of the compound represented by the above-mentioned formula (I) or (II) can be all used in the present invention, and each of the isomers may be used in an isolated form or a mixture thereof.

Furthermore, the compound represented by the above-mentioned formula (I) or (II) used in the present invention is capable of forming a derivative (prodrug), for instance, an ester, that can be readily hydrolyzed in a living body so that the desired effects can be exhibited. The preparation of the prodrug can be carried out according to a known method.

The above-mentioned isomers and derivatives may take the form of their salts.

The compound represented by the formula (I), the compound represented by the formula (II) and salts thereof, which are used as effective ingredients in the present invention have been found as the consequence of studies using the inhibitory activity for cancer cell (HL-60 cell) proliferation as an index. It is considered, therefore, that the effective ingredient essentially possesses an action such as apoptosis-inducing activity or anticancer activity. Concretely, the effective ingredient possesses regulating actions on the production of various biological factors and on expression of the genes encoding the factors, and possesses physiological activities such as inhibitory action of IL-6 production, inhibitory action of IL-10 production, inducting action of HO production, an inhibitory action of COX2 production, inhibitory action of MIP1α production, inhibitory action of RANTES production and inhibitory action of TNFα production. Among them, the effective ingredient is especially excellent in the various actions concretely exemplified herein as the physiological activities. It is deduced that these physiological activities are exhibited by the inhibition or induction of the gene transcriptional activation for various biological factors of which production is to be inhibited or induced and/or the inhibition or induction of their production from various cells. In addition, its action helps the sustenance and restoration of homeostasis in a living body and especially raises no concern about adverse reactions. These physiological activities are evident from Examples below. It has been confirmed that the inhibitory actions on the production of various biological factors are not associated with cytotoxic action.

According to the present invention, there is provided a suppressor for unbalance of gene expressions which modulates an unbalance of expressions of the genes for various biological factors exemplified herein based on these activities of the above-mentioned effective ingredient, and there is also provided a therapeutic agent or prophylactic agent for a disease that requires modulating an unbalance of expressions of the genes for various biological factors, comprising the above-mentioned effective ingredient. The therapeutic agent or prophylactic agent includes, for instance, a therapeutic agent or prophylactic agent for diseases that require inhibition of IL-6 production, those that require inhibition of IL-10 production, those that require induction of HO production, those that require inhibition of COX2 production, those that require inhibition of MIP1α production, those that require inhibition of RANTES production, those that require inhibition of TNFα production, and the like. The therapeutic agent or prophylactic agent can be used as an inhibitor of IL-6 production, an inhibitor of IL-10 production, an inducer of HO production, an inhibitor of COX2 production, an inhibitor of MIP1α production, an inhibitor of RANTES production, an inhibitor of TNFα production, and the like, and the therapeutic agent or prophylactic agent is suitably used as those concretely exemplified herein. Therefore, the present invention encompasses the use of the above-mentioned effective ingredient in the preparation of an inhibitor of IL-6 production, an inhibitor of IL-10 production, an inducer of HO production, an inhibitor of COX2 production, an inhibitor of MIP1α production, an inhibitor of RANTES production, or an inhibitor of TNFα production. The phrase "modulating an unbalance of gene expressions" as used herein refers to, but is not particularly limited to, that expression of a gene of which expression deviates from the normal level range due to disease or various exogenous factors is corrected so as to restore homeostasis in a living body. For instance, this term encompasses the inhibition of the expression level of a gene that exacerbates the pathologic conditions by increasing its expression level due to the disease, and the sustenance or enhancement of the expression level for a gene of which expression has been induced for the purpose of recovery from a disease. In other words, the suppressor for unbalance of gene expressions of the present invention refers to a suppressor for an unbalance of gene expressions associated with disease or various exogenous factors. Therefore, the suppressor for unbalance of gene expressions of the present invention can be used to ameliorate or prevent an unbalance of gene expressions. In other words, the suppressor for unbalance of gene expressions of the present invention contributes to the sustenance of homeostasis in a living body at the gene expression level, and can be referred to as a modulator of gene expressions disorder.

In a patient with atrial myxoma showing autoimmune disease-like symptoms, a large amount of IL-6 is produced by tumor cells. In addition, since the proliferation of myeloma cells derived from a multiple myeloma patient is inhibited by an anti-IL-6 antibody, IL-6 is highly likely to serve as a self-growth factor of myeloma cells. Furthermore, IL-6 is also contained in the urine from a patient with primary glomerulonephritis, and IL-6 functions as a growth factor for renal mesangium cells. It is considered that abnormal production of IL-6 is partially attributable to all the diseases exemplified herein. Therefore, the suppressor for unbalance of gene expressions of the present invention is useful in these diseases. By using the therapeutic agent or prophylactic agent of the present invention for a disease that requires modulating an unbalance of expressions for the IL-6 gene to inhibit the IL-6 production, the disease can be prevented or treated.

When IL-10 production is in excess, the production of interferon γ or the like is inhibited, thereby inhibiting immunity. Therefore, the suppressor for unbalance of gene expressions of the present invention is useful for a disease in which immunity is inhibited, partially due to excess production of IL-10, for instance, a disease that requires modulating an unbalance of expressions for the interferon γ gene (for instance, allergic disease such as asthma, pollenosis or atopic dermatitis). In addition, by using the therapeutic agent or prophylactic agent of the present invention for the disease that requires modulating of an unbalance of expressions for the IL-10 gene to inhibit the IL-10 production, the disease can be prevented or treated.

HO exists in two isozymes: 33 kDa HO1, and 36 kDa HO2. HO2 has a structure in which an additional amino acid sequence consisting of 20 amino acid residues is present at the N-terminal of HO1. Although the homology of the remaining portions is from 40 to 50%, the higher-order structures are similar. Both isozymes have a hydrophobic region at the C-terminal, where the isozymes bind to the microsome membrane. Since a soluble fraction with heme-degrading activity is obtained when microsome is treated with trypsin, the major domain including the active center appears to be projected toward the side of the cytoplasm.

HO1, an inducible enzyme, is induced remarkably in various cells by a chemical or physical factor such as a substrate heme, a heavy metal ion, a certain kind of an organic compound, hydrogen peroxide, heat shock, UV irradiation, or ischemia. HO2, a constitutive enzyme, is expressed in various tissues and is especially highly active in the brain and the testis.

HO degrades heme to biliverdin, CO and iron, and biliverdin is in turn further converted to bilirubin by a reductase. This bilirubin decreases various radicals. In other words, by inducing HO, the production of bilirubin having antioxidant activity is induced, so that various diseases caused by generation of radicals (for instance, various inflammatory diseases, diabetes mellitus, cancers, arteriosclerosis, neuropathy, ischemic reperfusion disorder) can be treated or prevented. Therefore, the suppressor for unbalance of gene expressions of the present invention is useful for these diseases. Also, by using the therapeutic agent or prophylactic agent of the present invention for a disease that requires modulating an unbalance of expressions for the HO gene to induce the HO production, the disease can be prevented or treated.

Arachidonic acid metabolism is significantly associated with the initiation of inflammation and ache in living tissues. COX2 is involved in arachidonic acid metabolism, and arachidonic acid derived from cell membrane phospholipids is metabolized by the action of COX2 to three substances: prostaglandin, prostacyclin and thromboxane. Accordingly, by using the suppressor for unbalance of gene expressions of the present invention to inhibit the COX2 production, analgesic and anti-inflammatory actions can be exhibited. In addition, by using the therapeutic agent or prophylactic agent of the present invention for a disease that requires modulating an unbalance of expressions for the COX2 gene to inhibit the COX2 production, the initiation of inflammation and ache in living tissue can be prevented or treated.

Chemokines, a class of cytokines showing specified chemotaxis for leukocytes, have a close association with allergic inflammation and autoimmune diseases. As mentioned above, chemokines are divided into two subfamilies: CXC chemokines and CC chemokines. Each chemokine consists of 92 to 99 amino acids and has four cysteine residues capable of forming disulfide bonding. In the CXC chemokine, there exists another amino acid intervening between the first two cysteine residues, and the CC chemokine has no such an intervening amino acid. The CXC chemokine exhibits chemotactic action mainly on neutrophil, and the CC chemokine exhibits chemotactic action on lymphocyte, monocyte, basophil and eosinophil.

RANTES, belonging to the CC chemokine, exhibits potent chemotaxis especially on eosinophil. Also, RANTES is produced from T cell, B cell, monocyte, fibroblast, macrophage, vascular endothelial cell, platelet, airway endothelial cell, and even eosinophil itself. RANTES is associated with degranulation, in addition to the potent chemotactic action on eosinophil. RANTES is a cytokine selectively causing memory T cell to migrate from helper (CD4⁺) T cell, thereby playing a key role in the initiation of allergic inflammation. In fact, there has been reported that RANTES is increased in its level in asthma, and that RANTES exists in teardrop in allergic conjunctivitis and the like. On the other hand, there has also been reported that RANTES enhances the active oxygen production by eosinophil. Therefore, by inhibiting the RANTES production, there can be expected prophylaxis and treatment of asthma and allergic inflammatory diseases partially due to the action of RANTES, for instance, allergic conjunctivitis, allergic dermatitis, allergic gastroenteritis, allergic vasculitis, allergic myelitis, allergic granulomatous angiitis, allergic encephalomyelitis, allergic rhinitis, allergic cystitis and the like. In addition, since the production of active oxygen by eosinophil is inhibited, there can be expected the exhibitions of an antioxidant effect and an anti-inflammatory effect in a living body. Therefore, the suppressor for unbalance of gene expressions of the present invention is useful for the above-mentioned diseases partially due to the action of RANTES, and also for the above-mentioned diseases exemplified as those that are effective in the induction of HO production. In addition, by using the therapeutic agent or prophylactic agent of the present invention for a disease that requires modulating an unbalance of expressions for RANTES gene to inhibit the RANTES production, the above-mentioned disease can be prevented or treated.

MIP1α, another CC chemokine, like RANTES, possesses the features of the CC chemokines. However, MIP1α is less chemotactic than that of RANTES. In patients with bronchial asthma, for instance, RANTES and MIP1α show different expression patterns. It has been reported that MIP1α increases its level prior to exacerbation of the attack after the treatment, whereas RANTES increases its level from early after the attack. Therefore, it is considered that the exacerbation of asthma and allergic inflammatory diseases as those exemplified above can be inhibited by inhibiting the MIP1α production. In other words, the suppressor for unbalance of gene expressions of the present invention is especially useful for inhibition of the exacerbation of such diseases. By using the therapeutic agent or prophylactic agent of the present invention for a disease that requires modulating an unbalance of expressions for the MIP1α gene to inhibit the MIP1α production, the exacerbation of the disease can be prevented or treated.

Since an agaro-oligosaccharide DGE described in Examples set forth below, which is one effective ingredient used in the present invention, is capable of modulating the unbalance of expressions for both the RANTES and MIP1α genes, it is considered to be effective in the prevention and treatment of allergic inflammatory diseases, and even in the inhibition of the exacerbation of the pathologic conditions during the treatment.

TNFα, another kind of a cytokine, is considered to directly cause inflammation associated with autoimmune diseases such as rheumatoid arthritis, and with inflammatory diseases.

TNFα was found as a factor that induces hemorrhagic necrosis at tumoral portions, and it is presently recognized as a cytokine involved widely in the inflammation-based defense and immune mechanisms of a living body, and a disruption of the TNFα production regulatory mechanism leads to various undesirable events in the host. Concretely, excessive or unregulated production of TNFα is associated with a wide variety of diseases, including rheumatoid arthritis, rheumatic myelitis, arthritis deformans, gouty arthritis, sepsis, septic shock, endotoxin shock, gram-negative bacterial sepsis, toxic shock syndrome, cerebral malaria, chronic pneumonia, graft-versus-host reaction, homograft rejection, fever and myalgia due to infection like influenza, cachexia secondary to infection or malignant tumor, cachexia secondary to human acquired immunodeficient syndrome (AIDS), AIDS, AIDS-related syndrome, keloid formation, ulcerative colitis, multiple sclerosis, and autoimmune diseases including autoimmune diabetes mellitus and systemic lupus erythematosus *[Molecular Medicine,* **33**, 1010-1020, 1182-1189 (1996)].

Therefore, the suppressor for unbalance of gene expressions of the present invention is useful for diseases that are mediated or exacerbated by TNFα as exemplified above. By using the therapeutic agent or prophylactic agent of the present invention for a disease that requires modulating an unbalance of expressions for the TNFα gene to inhibit the TNFα production, the disease can be prevented or treated. For instance, by using the suppressor for unbalance of gene expressions or the like of the present invention, the symptoms of inflammation and rheumatism, especially rheumatoid arthritis, are ameliorated, the levels of inflammation markers C-reactive protein (CRP), rheumatoid factor (RF), and erythrocyte sedimentation rate (ESR) are decreased dramatically, and complication symptoms such as dysbasia are ameliorated remarkably.

Furthermore, the above-mentioned effective ingredient used in the present invention acts to modulate an unbalance of gene expressions for each of other various biological factors that are important to the sustenance of homeostasis in a living body.

Organisms are susceptible to be under various forms of stress from exogenous factors such as environmental changes and endogenous factors such as viral or bacterial infections. The forms of stress include, for instance, high-temperature and low-temperature environments, starvation, exposure to a chemical substance, and the like. For instance, the chemical substance includes tetradecanoylphorbol acetate (TPA), lipopolysaccharide (LPS), phytohemagglutinin (PHA), okadaic acid and the like. TPA and LPS, in particular, are used for the purposes of artificially preparing an inflammation model utilizing its action that causes inflammation when administered to a living body.

As a result of being susceptible to be under the form of stress described above, expression of various genes is altered in a living body. Among them, there are some genes in which expression is induced or enhanced by stress, thereby causing adverse affect to a living body (group A), and other genes in which expression is inhibited, thereby interfering with the normal biological activities of a living body (group B). A persistence of an unbalance of gene expressions for each of the above gene groups under stress will lead to the onset of various diseases. Therefore, restoration of the changes in gene expressions under stress to the normal state leads to the treatment or prevention of various diseases.

In other words, according to the suppressor for unbalance of gene expressions or the like of the present invention, an unbalance of expressions for the gene for each of various biological factors that may cause an unbalance of gene expressions under some stress, including the above-mentioned cytokines and various enzymes, can be restored to the normal conditions (healthy state in which homeostasis is sustained).

For instance, the genes (groups A and B) of which unbalance of gene expressions can be modulated to the normal state by DGE described in Examples set forth below, which can be used as effective ingredients of the present invention, include the genes concretely listed in Tables 1 and 2.

**Table 1**

| Name of Genes (Abbreviation) | GenBank Accession No. |
|---|---|
| - Cytokine and Cytokine Receptor | |
| interleukin-6 (IL-6) | M14584 |
| interleukin-1α (IL-1α) | X02851 |
| interleukin-1β (IL-1β) | K02770 |
| tumor necrosis factor α (TNFα) | M10988 |
| interleukin-7 receptor (IL-7r) | M29696 |

| - CC Chemokine | |
|---|---|
| macrophage inflammatory protein-1-β (MIP1β) | J04130 |
| liver and activation-regulated chemokine (LARC) | D86955 |
| macrophage-derived chemokine (MDC) | U83171 |

| - CXC Chemokine | |
|---|---|
| macrophage inflammatory protein-2-β (MIP2β) | M36821 |
| macrophage inflammatory protein-2-α (MIP2α) | M36820 |
| growth regulated protein-1 (Gro1) | J03561 |

**Table 2**

| Name of Genes (Abbreviation) | GenBank Accession No. |
|---|---|
| - Others | |
| matrix metalloproteinase-9 (MMP-9) | J05070 |
| migration inhibitory factor-related protein-8 (MRP8) | X06234 |
| lyzozyme (Lyz) | M21119 |
| GABA(A) receptor-associated protein (GABArp) | NM007278 |
| interferon-induced 17-kDa/15-kDa protein | M13755 |
| (IFNp15/p17) | |
| interferon-inducible protein p78 (IFNp78) | M33882 |
| SCO (cytochrome oxidase deficient, yeast) | AL021683 |
| homolog-2 (SCO2) | |
| transketolase (TK) | L12711 |
| adenosine A2a receptor (ADORA2A) | S46950 |
| CD37 antigen (CD37) | X14046 |
| properdin P factor, complement (PFC) | M83652 |
| regulator of G-protein signaling-2 (RGS2) | L13463 |
| Nef-associated factor-1 (NAF1) | AJ011896 |
| myeloid leukemia cell differentiation protein-1 (MCL1) | L08246 |
| signal peptidase complex (18 kDa) (SPC18) | AF061737 |

Especially according to the present invention, there is provided a therapeutic agent or prophylactic agent which is suitable for an inflammatory disease that requires modulating an unbalance of expressions for one or more genes selected from the genes for the various biological factors exemplified in the above-mentioned Tables 1 and 2, comprising the above-mentioned effective ingredient.

The suppressor for unbalance of gene expressions of the present invention may be the above-mentioned effective ingredient *per se,* or a composition comprising the above-mentioned effective ingredient. For instance, the suppressor for unbalance of gene expressions of the present invention can be prepared by mixing the above-mentioned effective ingredient and another ingredient that can be used for the same purpose by a conventional method. The content of the effective ingredient in the suppressor is not particularly limited, as long as the amount is adjusted such that the effective ingredient can be administered preferably within the dose range described below in consideration of its method of administration and the like. In addition, the therapeutic agent or prophylactic agent of the present invention comprises the above-mentioned effective ingredient, and may be formed into a preparation by combining the effective ingredient with a known pharmaceutical vehicle. The preparation is generally produced by formulating the effective ingredient with a pharmaceutically acceptable liquid or solid vehicle, and optionally adding a solvent, a dispersant, an emulsifier, a buffer, a stabilizer, an excipient, a binder, a disintegrant, a lubricant, or the like, and forming the resulting mixture into a solid agent such as a tablet, a granule, a powder, a fine powder, or a capsule, or a liquid agent such as a general liquid agent, a suspension agent, or an emulsion agent. In addition, a dry product which can be made into a liquid form by adding an appropriate vehicle immediately before use can be also produced.

The therapeutic agent or prophylactic agent of the present invention can be administered as an oral preparation, a non-oral preparation such as an injection or a drip infusion, or an external preparation.

The pharmaceutical vehicle can be selected according to the above-mentioned dosage form and preparation form. In the case of an oral preparation as a solid composition, the oral preparation can be produced in the form of a tablet, a pill, a capsule, a powder, a fine granule, a granule, and the like, utilizing starch, lactose, white sugar, mannitol, carboxymethyl cellulose, corn starch, inorganic salts, and the like. In addition, in the production of an oral preparation, there can also be formulated a binder, a disintegrant, a surfactant, a lubricant, a fluidity accelerator, a taste corrective, a colorant, a perfume, or the like. In the case of forming into a tablet or pill, for instance, the tablet or pill may be covered with a sugar-coating such as sucrose, gelatin or hydroxypropyl cellulose, or with a film made of a substance soluble in the stomach or intestine as occasion demands. In the case of an oral preparation as a liquid composition, the oral preparation can be prepared in the form of a pharmaceutically acceptable emulsion, solution, suspension, syrup, or the like, using purified water, ethanol, or the like, for instance, as a vehicle. Furthermore, an auxiliary agent such as a wetting agent or a suspending agent, a sweetener, a flavor, an antiseptic, or the like may be added as desired.

On the other hand, a non-oral preparation is prepared by dissolving or suspending the above-mentioned effective ingredient of the present invention in a diluent such as distilled water for injection, physiological saline, an aqueous solution of glucose, vegetable oil for injection, sesame oil, peanut oil, soybean oil, corn oil, propylene glycol or polyethylene glycol, by a conventional method, and adding a microbicide, a stabilizer, an osmotic regulator, a soothing agent, or the like as necessary. It is also possible to produce a solid composition and dissolve it in sterile water or a sterile solvent for injection before use.

The external preparation includes solid to semi-solid or semi-solid to liquid preparations for percutaneous administration or transmucosal (oral or intranasal) administration. The external preparation also includes suppositories and the like. For instance, the external preparation may be prepared as liquid preparations including emulsions, suspensions such as lotions, external tinctures, and liquids for transmucosal administration; ointments including oily ointments and hydrophilic ointments; medical adhesives for percutaneous administration or transmucosal administration such as films, tapes and poultices; and the like.

Each of the above-mentioned various preparations can be appropriately produced by conventional methods by utilizing known pharmaceutical vehicles. The content of the effective ingredient in the preparation is the same as that in the above-mentioned suppressor for unbalance of gene expressions.

For instance, the above-mentioned suppressor for unbalance of gene expressions or the like can be administered via an appropriate route according to each dosage form. The method of administration is not particularly limited, and the administration can be made orally, externally and via injection. The injection can, for instance, be administered intravenously, intramuscularly, subcutaneously, intradermally, or the like, and the external preparation includes suppositories and the like.

In addition, the dose is changeable and properly set depending upon its preparation form, administration method, purpose of use, age, body weight, symptom or the like of a mammal (for instance, a patient) to which the agent is applied, or the like. The dose for adult per day of the above-mentioned effective ingredient is preferably from 10 µg to 200 mg/kg. When the effective ingredient is administered as a preparation, the preparation may be administered so that the dose of the effective ingredient falls within the above-mentioned preferable range. As a matter of course, the dose varies depending upon various conditions, so that an amount smaller than the dose mentioned above may be sufficient, or an amount exceeding the dose range may be required. Dosing may be carried out once or in two or more divided portions in a day within the desired dose range. In addition, as the method of oral administration, the above-mentioned suppressor for unbalance of gene expressions or the *like per se* may be administered orally. Alternatively, the desired effects of the present invention can be obtained by adding optionally the above-mentioned effective ingredient to a foodstuff to be taken on a daily basis.

In addition, as one embodiment of the present invention, there is provided a method for modulating an unbalance of gene expressions comprising administering the above-mentioned effective ingredient to a mammal. Especially, this method can be suitably used for modulating an unbalance of expressions for at least one gene selected from the genes exemplified above for the various biological factors, preferably the above-mentioned 31 kinds of a group of genes. This method can be carried out by administering the above-mentioned effective ingredient, preferably in the form as a suppressor for unbalance of gene expressions or the like of the present invention, to a mammal that is predicted to require or requires modulating the unbalance of gene expressions. By administering the effective ingredient, the production of the desired biological factor is induced or inhibited, so that homeostasis in a living body is restored. The administration method, dose, or the like of the effective ingredient may be determined in accordance with the above-mentioned suppressor for unbalance of gene expressions or the like. In this method, the above-mentioned suppressor and the like are suitably used as inducers or inhibitors of the production of various factors.

Further, in another embodiment of the present invention, there can be provided a screening method for a therapeutic agent or prophylactic agent for a disease involving various biological factors, for instance, cytokines or enzymes. The screening method can be carried out by, for instance, evaluating whether or not the test substance modulates the expression level of at least one gene selected from the genes encoding the various biological factors exemplified above, preferably from the above-mentioned 31 kinds of a group of genes, in a treatment that changes the expression level of the gene.

Incidentally, no case of death is found even when any of the above-mentioned effective ingredients used in the present invention is orally administered to a rat in a single dose of 100 mg/kg.

### EXAMPLES

The present invention will be more concretely described below by means of Examples, without limiting the present invention thereto.

### Preparation Example 1 Preparation of L-Glycero-1,5-epoxy-1αβ,6-dihydroxy-cis-hex-3-en-2-one (DGE) and D-Glycero-1,5-epoxy-1αβ,6-dihydroxy-cis-hex- 3-en-2-one (κ-DGE)

The amount 2.5 g of a commercially available agar (Agar Noble) was suspended in 50 ml of 0.1 N HCI, and the suspension was heated at 100°C for 13 minutes to dissolve the agar. After cooling the solution to room temperature, the pH was adjusted with NaOH to 12, and thereafter the solution was neutralized.

The neutralized solution was subjected to the following normal phase HPLC. Each peak fraction was collected and dried under reduced pressure, and the obtained solid was then dissolved in water. The inhibitory activity for cancer cell proliferation of each fraction was assayed using HL-60 cells. There was confirmed inhibitory activity for cancer cell proliferation in a fraction having a retention time of from 4.05 to 4.16 minutes.

Next, this fraction having a retention time of from 4.05 to 4.16 minutes was collected in a large amount, and subjected to structural analysis. It was confirmed that this fraction is L-glycero-1,5-epoxy-1αβ,6-dihydroxy-*cis*-hex-3-en-2-one (DGE). The conditions for normal phase HPLC are given hereinbelow:
Column: PALPAK Type S (4.6 mm × 250 mm, manufactured by Takara Shuzo Co., Ltd.)
Mobile phase A: 90% aqueous acetonitrile solution
Mobile phase B: 50% aqueous acetonitrile solution
Flow rate: 1 ml/minute
Elution: mobile phase A (10 minutes) → linear concentration gradient from mobile phase A to mobile phase B (40 minutes) → mobile phase B (10 minutes)
Detection: absorbance at 195 nm
Column temperature: 40°C

Similarly, κ-carrabiose was treated with an alkali in accordance with the above-mentioned case of agar. D-Glycero-1,5-epoxy-1αβ,6-dihydroxy-*cis*-hex-3-en-2-one (κ-DGE) was similarly purified from the resulting alkali-treated product.

Here, κ-carrabiose was prepared as follows. Concretely, 2.5 g of κ-carrageenan (manufactured by Sigma, C-1263) was suspended in 50 ml of 0.1 N HCl, and the suspension was heated at 100°C for 16 minutes to dissolve the carrageenan. The solution was cooled to room temperature, and the pH was adjusted with NaOH to near neutrality. Thereafter, the solution was filtered with Cosmonise Filter S (manufactured by nakalai tesque), and separated by the following normal phase HPLC. κ-Carrabiose having a retention time of 27.8 minutes was collected, and dried under reduced pressure, to give κ-carrabiose. The conditions for normal phase HPLC are given hereinbelow:
Column: PALPAK Type S (4.6 mm × 250 mm, manufactured by Takara Shuzo Co., Ltd.)
Mobile phase A: 90% aqueous acetonitrile solution
Mobile phase B: 50% aqueous acetonitrile solution
Flow rate: 1 ml/minute
Elution: mobile phase A (10 minutes) → linear concentration gradient from mobile phase A to mobile phase B (40 minutes) → mobile phase B (10 minutes)
Detection: absorbance at 195 nm
Column temperature: 40°C

### Preparation Example 2 Preparation of 5-L-Glutathion-S-yl-2-hydroxy-3,7-dioxabicyclo[2.2.2]octan-1-ol (DGE-GSH)

Each of DGE and reduced glutathione (GSH, manufactured by nakalai tesque) was dissolved in PBS so as to have a concentration of 20 mM, and reacted at 37°C overnight. One-hundred microliters of this reaction solution was fractionated on normal phase column PAL-PAK Type S. The normal phase chromatography was carried out under the conditions of a flow rate of 1 ml/minute, 90% aqueous acetonitrile solution for 0 to 10 minutes, a linear concentration gradient from 90% aqueous acetonitrile solution to 50% aqueous acetonitrile solution for 10 to 50 minutes, and a detection at 195 nm, and fractions were collected every 1.5 minutes. Each of the fractions was concentrated to dryness, and thereafter re-dissolved in 50 µl of distilled water. The inhibitory activity for cancer cell proliferation of each fraction was assayed using HL-60 cells. As a result, the cell proliferation was inhibited in fractions near fraction nos. 30 to 40. Therefore, 50 µl of similarly prepared active fractions were further fractionated by reverse phase chromatography (TSK gel ODS-80Ts (5 µm), manufactured by Tosoh Corporation, 4.6 × 250 mm). The chromatography was carried out under the conditions of a flow rate of 1 ml/minute, 0.1% TFA-containing distilled water for 0 to 15 minutes, a linear concentration gradient from 0.1% TFA-containing distilled water to 0.1% TFA-containing 50% aqueous acetonitrile solution for 15 to 30 minutes, 0.1% TFA-containing 50% aqueous acetonitrile solution for 30 to 45 minutes, and a detection at 215 nm, and fractions were collected every 1.5 minutes. Each of the fractions was concentrated to dryness, and thereafter re-dissolved in 50 µl of distilled water. The inhibitory activity for cancer cell proliferation of each fraction was assayed using HL-60 cells. As a result, there was confirmed the inhibitory activity for cancer cell proliferation in fractions having a retention time of near 4 to 9 minutes.

Therefore, the above-described normal chromatography was repeated 10 times. Each of its active fractions was collected, concentrated to dryness, and re-dissolved in 1 ml of distilled water, to prepare a purified fraction by normal phase chromatography. Next, the fraction purified by normal chromatography was further subjected to the above-described reverse phase chromatography, and its active fraction was collected and concentrated to dryness. As a result, 5 mg of an active compound was obtained from a 1 ml reaction solution containing 20 mM DGE and 20 mM glutathione. This compound was structurally determined, and it was confirmed that the compound is 5-L-glutathion-S-yl-2-hydroxy-3,7-dioxabicyclo[2.2.2]octan-1-ol (hereinafter simply referred to "DGE-GSH").

### Example 1

Four milliliters of a 10% fetal bovine serum-containing RPMI 1640 medium (manufactured by Bio Whittaker, 12-702F) was intraperitoneally injected to ddy mouse (Nippon SLC, female, 7-weeks old), and the mouse was well massaged, and the medium was taken out to obtain peritoneal cells. The peritoneal cells were suspended in the 10% fetal bovine serum-containing RPMI 1640 medium so as to have a concentration of 10⁶ cells/ml, and dispensed into 48-well microtiter plate in a volume of 500 µl each. The peritoneal cells were cultured at 37°C for 2 hours in the presence of 5% CO₂ gas. Thereafter, the culture supernatant was removed to give adherent cells to be used as peritoneal macrophage. To each well was freshly dispensed a Dulbecco's modified Eagle's medium (manufactured by Bio Whittaker; 12-917F) without phenol red containing 10% fetal bovine serum and 2 mM L-glutamine in a volume of 500 µl. To each well was added 5 µl of each of 2 mM and 1 mM DGE aqueous solutions (final concentrations of 20 µM and 10 µM, respectively), and 5 µl of 10 µg/ml 12-o-tetradecanoylphorbol 13-acetate (TPA, manufactured by Gibco, 13139-019) aqueous solution was added thereto, and the cells were cultured for additional 14 hours, and thereafter the culture supernatant was collected. The content of interleukin-6 (IL-6) in the culture supernatant was assayed by enzyme immuno-sandwich assay (ELISA: Matched antibody Pair SamplePak mouse IL-6, manufactured by Endogen). Here, the group with no addition of DGE and TPA aqueous solution (control) was set as a negative control, and the group with addition of TPA aqueous solution alone (control + TPA) was set as a positive control. In addition, all of the assays were carried out twice.

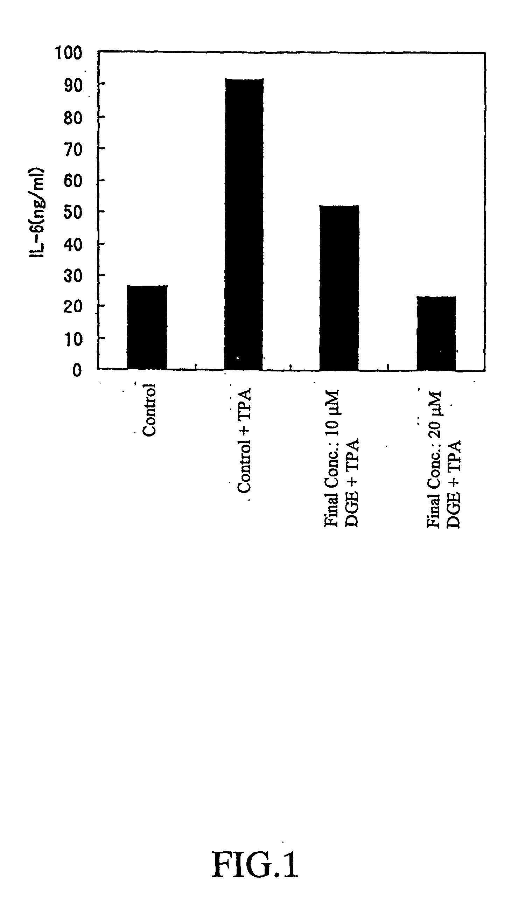

As a result, the inhibition of TPA-induced IL-6 production was found DGE-concentration dependently in the group with addition of DGE. The results are shown in Figure 1. Concretely, Figure 1 is a graph showing a concentration of IL-6 in the culture supernatant when the cells were cultured under each of the culture conditions, wherein the axis of abscissas is culture conditions, and the axis of ordinates is the concentration of IL-6 (ng/ml).

In addition, tests were carried out in the same manner for κ-DGE and DGE-GSH to confirm an inhibitory action for IL-6 production.

### Example 2

RAW 264.7 cells (ATCC TIB 71) were suspended in a Dulbecco's modified Eagle's medium (manufactured by Bio Whittaker; 12-604F) containing 10% fetal bovine serum (manufactured by Gibco), so as to have a concentration of 3 × 10⁵ cells/ml. The cell suspension was added to each well of a 48-well microtiter plate in a volume of 0.5 ml each. The cells were cultured at 37°C in the presence of 5% CO₂ gas overnight. To each well was added 5 µl of a 2 mM DGE aqueous solution (final concentration: 20 µM), and the cells were cultured for additional 5 hours. Thereafter, 5 µl of a 100 µg/ml lipopolysaccharide (LPS, manufactured by Sigma, L-2012) aqueous solution was added thereto, and the cells were cultured for 18 hours, and thereafter the culture supernatant was recovered. The content of the interleukin-10 (IL-10) in the culture supernatant was assayed by the enzyme immuno-sandwich assay. Here, the group with no addition of DGE and LPS aqueous solution (control) was set as a negative control, and the group with addition of LPS aqueous solution (control + LPS) alone was set as a positive control. In addition, all of the assays were carried out twice.

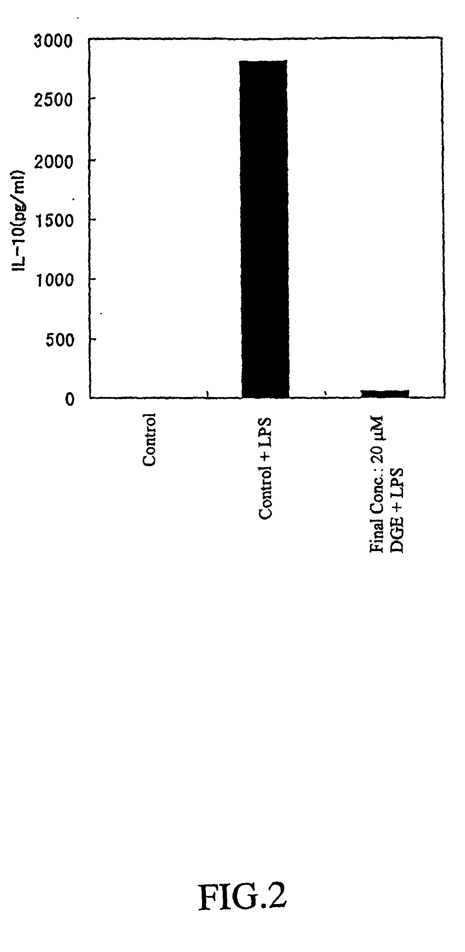

As a result, the inhibition of LPS-induced IL-10 production was found in the group with addition of DGE. The results are shown in Figure 2. Concretely, Figure 2 is a graph showing a concentration of IL-10 in the culture supernatant when the cells were cultured under each of the culture conditions, wherein the axis of abscissas is culture conditions, and the axis of ordinates is the concentration of IL-10 (pg/ml).

In addition, tests were carried out in the same manner for κ-DGE and DGE-GSH to confirm an inhibitory action for IL-10 production.

### Example 3

RAW 264.7 cells (ATCC TIB 71) were suspended in a Dulbecco's modified Eagle's medium (manufactured by Bio Whittaker; 12-604F) containing 10% fetal bovine serum (manufactured by Gibco), so as to have a concentration of 3 × 10⁵ cells/ml. The cell suspension was added to each well of a 6-well microtiter plate in a volume of 5 ml each. The cells were cultured at 37°C in the presence of 5% CO₂ gas overnight. To each well was added 50 µl of 4 mM, 2 mM or 1 mM DGE aqueous solution (each of final concentrations: 40 µM, 20 µM or 10 µM), and the cells were cultured for 15 hours. Here, the group with addition of 5 µl of 3 mM 15-deoxy-Δ12,14-prostaglandin J₂ (manufactured by Cayman Chemical, 18570) dimethyl sulfoxide solution was set as a negative control for HO1 induction. The group with addition of water (here, the induction action for HO1 production was not found in dimethyl sulfoxide) was set as a positive control. The cells were harvested by peeling off the cells from the plate with a scraper. The harvested cells were suspended in 0.1 M Tris-HCl buffer (pH 7.5) containing 0.05 mM pepstatin (manufactured by Sigma, P5318), 0.2 mM leupeptin (manufactured by Sigma, L2884), 1mM phenylmethylsulfonyl fluoride (manufactured by nakalai tesque, 273-27), 10 mM disodium dihydrogenethylenediaminetetraacetate and 0.1% Triton X-100, and subjected to freezing and thawing once. Thereafter, the supernatant obtained by centrifugation was used as a protein fraction. The protein content in the protein fraction was assayed by Micro BCA Protein Assay Reagent (one manufactured by Pierce sold by Takara Shuzo Co., Ltd., P7411). Each of the resulting protein fraction samples was mixed with an equal volume of 0.125 M Tris-HCl buffer (pH 6.8) containing 4% sodium lauryl sulfate (SDS), 2% 2-mercaptoethanol, 0.001% Bromophenol Blue and 20% glycerol, and the mixture was treated at 100°C for 5 minutes. A mixture containing a protein in an amount of 10 µg or so was loaded on 12.5% SDS-polyacrylamide gel, and electrophoresed with a constant current of 20 mA. In a blotting buffer containing 48 mM Tris, 39 mM glycine, 20% methanol, 0.0375% SDS, the gel after the electrophoresis was transferred to a PDVF membrane (manufactured by Millipore Corporation, IPVH000 10) in accordance with the attached protocol, using transblot SD cell semi-drive blotting device (manufactured by BioRad), at a constant voltage of 15 V for 25 minutes. The transferred PVDF membrane was blocked overnight in a Block Ace (manufactured by DAINIPPON PHARMACEUTICAL CO., LTD., UK-B25) solution at 4°C. The blocked membrane was washed for 15 minutes thrice with phosphate-buffered physiological saline containing 0.1% Tween 20 under gentle shaking. Thereafter, the membrane was subjected to the reaction under gentle shaking in phosphate-buffered physiological saline containing 10% Block Ace with a 200 ng/ml anti-HO1 antibody (N-19, manufactured by Santa Cruz, sc-7696) and 0.1% Tween 20 at room temperature for 1 hour, and was washed for 15 minutes thrice with phosphate-buffered physiological saline containing 0.1% Tween 20 under gentle shaking. Next, the membrane was subjected to the reaction under gentle shaking in phosphate-buffered physiological saline with 10% Block Ace with 0.1% peroxidase-conjugated rabbit anti-goat IgG (H + L) antibody (manufactured by Zymed, 61-1620) and 0.1% Tween 20 at room temperature for 1 hour, and was washed for 15 minutes five times with phosphate-buffered physiological saline containing 0.1% Tween 20 under gentle shaking. Subsequently, a PVDF membrane was stained by using Western blot Chemiluminescence Reagent Plus (one manufactured by NEN Life Science Products sold by Daiichikagaku, NEL103) in accordance with the attached protocol, and sensitized on an X-ray film (manufactured by Kodak, CAT 165 1454). The sensitized film was developed with FPM800 (manufactured by Fuji Photo Film Co., Ltd.).

As a result, there could be confirmed a band ascribed to HO1 protein in the group with addition of DGE. In addition, the intensity of the band was DGE concentration-dependent. The results are shown in Table 3. In the table, + symbols were given in accordance with the intensity of the band for HO1 protein. In other words, in Table 3, those showing no bands are indicated by "-," and the intensities of the bands are indicated by +-, +, ++ in the ascending order of the intensities of the bands.

**Table 3**

| Sample | Intensity of Bands of HO1 Protein |
|---|---|
| Water (Negative Control) | - |
| Final Concentration 40 µM DGE | ++ |
| Final Concentration 20 µM DGE | + |
| Final Concentration 10 µM DGE | +- |
| 15-Deoxy-Δ12,14 prostaglandin J₂ (Positive Control) | ++ |

In addition, tests were carried out in the same manner for κ-DGE and DGE-GSH to confirm an induction action for HO production.

### Example 4

### (1) Isolation and Storage of PBMCs

Four-hundred milliliters of blood was collected from a human normal individual donor. The collected blood was diluted 2-folds with PBS(-), overlaid on Ficoll-paque (manufactured by Pharmacia), and centrifuged at 500 × g for 20 minutes. The peripheral blood mononuclear cells (PBMCs) in the intermediate layer was collected with a pipette, and washed with RPMI 1640 medium (manufactured by Bio Whittaker). The collected PBMCs was suspended in a storage solution of 90% FCS (manufactured by JRH Biosciences)/10% dimethyl sulfoxide, and stored in liquid nitrogen. During the experiment, these stored PBMCs were rapidly melted in water bath at 37°C, and washed with RPMI 1640 medium containing 10 µg/ml Dnase (manufactured by Calbiochem). Thereafter, the number of living cells was calculated by trypan blue staining method, and subjected to each experiment.

### (2) Isolation of Monocytes

PBMCs were suspended in RPMI 1640 medium containing 5% human serum of blood type-AB, 0.1 mM nonessential amino acid, 1 mM sodium pyruvate, 2 mM L-glutamine (hereinabove manufactured by Bio Whittaker), 10 mM HEPES (manufactured by nakalai tesque), and 1% streptomycin-penicillin (manufactured by Gibco BRL) (hereinafter simply referred to as "5HRPMI medium") so as to have a concentration of 4 × 10⁶ cells/ml. Thereafter, the suspension was spread on a 6-well cell culture plate (manufactured by Falcon) in a volume of 3 ml/well, and the cells were incubated in a wet-type incubator at 37°C and 5% CO₂ for 1.5 hours. After 1.5 hours, the non-adherent cells were removed by suction, and each well was washed with RPMI 1640 medium, and 2 ml of the 5HRPMI medium was added to the wells, to give monocytes.

### (3) Preparation of RNA

To the monocytes obtained above was added in each well 8 µl of an aqueous DGE solution, or 8 µl of water as a control, and the cells were incubated in a wet-type incubator at 37°C and 5% CO₂ for 5 hours. Thereafter, with addition of 2 µl of a 1 µg/ml aqueous LPS solution or 2 µl of a 25 µM TPA dimethyl sulfoxide solution to each well, or with addition of neither of these substances thereto, the cells were cultured for additional 4 hours. Thereafter, RNA was prepared from each cell in accordance with the description of the kit using RNeasy Mini Kit (manufactured by QIAGEN, 74104). The group with addition of water alone or the aqueous DGE solution alone to the cells was set as a negative control, and the group with addition of water and the aqueous LPS solution or the aqueous TPA solution to the cells was set as a positive control.

### (4) Synthesis of cDNA

A liquid mixture having a total volume of 60 µl of 0.5 µg of RNA prepared above, 10 mM Tris-HCl (pH 8.3), 50 mM KCl, 5 mM MgCl₂, 1 mM dNTP mixtures, 150 pmol of Random 6mers, 60 U Ribonuclease Inhibitor (manufactured by Takara Shuzo Co., Ltd., 2310A), and 15 U Reverse Transcriptase XL (AMV) (manufactured by Takara Shuzo Co., Ltd., 2620A) were incubated at 30°C for 10 minutes and thereafter at 42°C for 1 hour using a thermal cycler (GeneAmp PCR System 9600, manufactured by Applied Biosystems), and the mixture was heated at 99°C for 5 minutes to deactivate the enzyme, to give cDNA.

### (5) Synthesis of Primers

Oligonucleotide primers for reaction of PCR were designed based on nucleotide sequence of COX2 mRNA [GenBank Accession NO: AL033533], and synthesized by using a DNA synthesizer (manufactured by Applied Biosystems). Concretely, each of oligonucleotide primers CX1 (SEQ ID NO: 1 of Sequence Listing, hereinafter referred to the same) and CX2 (SEQ ID NO: 2) was synthesized. Each of oligonucleotide primers MP1 (SEQ ID NO: 3) and MP2 (SEQ ID NO: 4) was synthesized based on nucleotide sequence of MIP1α mRNA [GenBank Accession NO: M23452]. Each of oligonucleotide primers RS1 (SEQ ID NO: 5) and RS2 (SEQ ID NO: 6) was synthesized based on nucleotide sequence of human RANTES mRNA [GenBank Accession NO: NM 002985]. Further, as an internal standard, each of oligonucleotide primers AC1 (SEQ ID NO: 7) and AC2 (SEQ ID NO: 8) was synthesized based on nucleotide sequence of human β-actin mRNA [GenBank Accession NO: M10277].

### (6) DNA Fragment Amplification by PCR Method with cDNA as Template

A reaction system having a total volume of 25 µl of 5 pmol each of the synthesized oligonucleotide primers CX1 and CX2, or MP1 and MP2, or RS1 and RS2, and the internal standard oligonucleotide primers AC1 and AC2 of human β-actin, 2.5 µl of the solution of cDNA prepared above, 2.5 µl of 10 × Ex Taq buffer (manufactured by Takara Shuzo Co., Ltd.), 0.625 U TaKaRa Ex Taq polymerase (manufactured by Takara Shuzo Co., Ltd., RR001A) and 0.2 mM dNTP mixtures was reacted using the thermal cycler according to a program of one cycle of 94°C for 2 minutes, 30 cycles of 95°C for 30 seconds, 59°C for 30 seconds, and 73°C for 30 minutes, and one cycle of 72°C for 5 minutes. The reacted sample was frozen at -20°C and stored until the sample was analyzed.

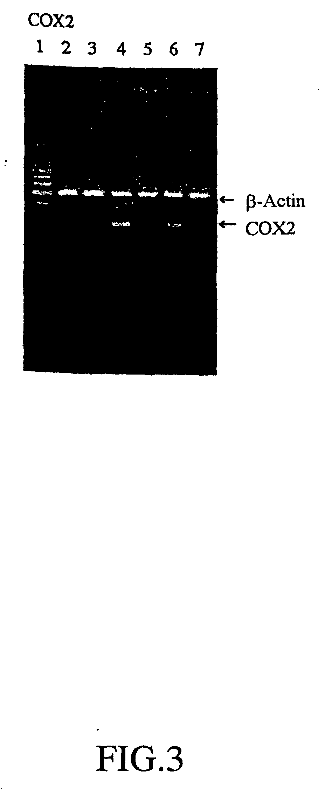

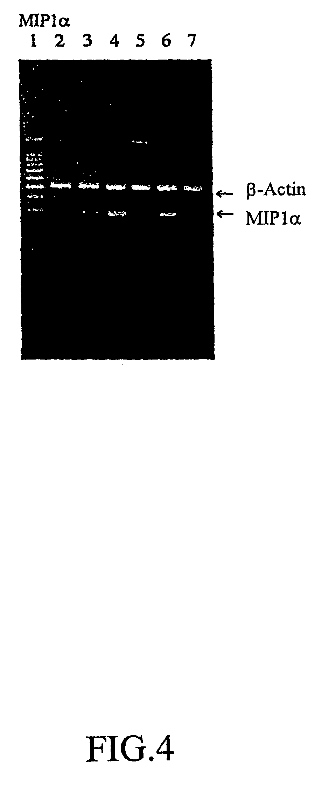

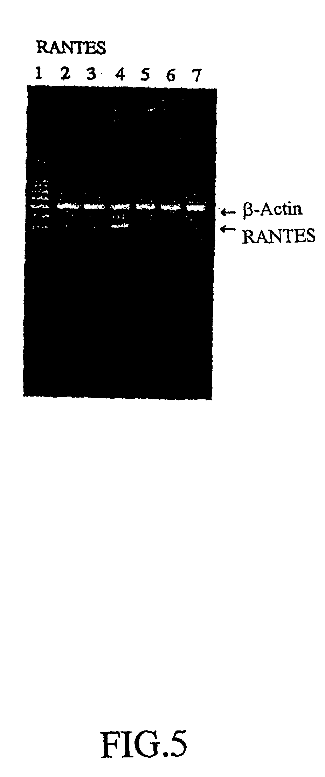

Ten microliters of each PCR mixture was analyzed by 2.0% agarose gel electrophoresis. As a result, amplification of the bands ascribed to each of COX2, MIP1α, and RANTES mRNA could be confirmed in the group with addition of LPS alone. Concretely, there could be confirmed the induction of these genes by LPS. By contrast, amplification of the bands ascribed to any of COX2, MIP1α, and RANTES mRNA could not be confirmed in the group with addition of DGE and LPS. In other words, it was clarified that the induction of these genes by LPS was inhibited by DGE. Also, amplification of the bands ascribed to each of COX2 and MIP1α mRNA could be confirmed in the group with addition of TPA alone. In other words, there could be confirmed the induction of these genes by TPA. By contrast, amplification of the bands ascribed to any of COX2 and MIP1α mRNA could not be confirmed in the group with addition of DGE and TPA. In other words, it was clarified that the induction of these genes by TPA was inhibited by DGE. The results are shown in Figures 3 to 5. Concretely, the results of agarose gel electrophoresis analysis of a reaction mixture obtained by PCR amplification of cDNA derived from each mRNA of COX2 are shown in Figure 3, those of MIP1α are shown in Figure 4 and those of RANTES are shown in Figure 5. In addition, each lane of Figures 3 to 5 is as follows: Lane 1 is 100 bp DNA ladder marker, lane 2 control (water), lane 3 the group with addition of DGE, lane 4 the group with addition of LPS, lane 5 the group with addition of DGE and LPS, lane 6 the group with addition of TPA, and lane 7 the group with addition of DGE and TPA.

### Example 5

### (1) Isolation of Monocytes

PBMCs were isolated in the same manner as in item (1) of Example 4. PBMCs were suspended in RPMI 1640 medium containing 5% human serum of blood type-AB, 0.1 mM nonessential amino acid, 1 mM sodium pyruvate, 2 mM L-glutamine (hereinabove manufactured by Bio Whittaker), 10 mM HEPES (manufactured by nakalai tesque), and 1% streptomycin-penicillin (manufactured by Gibco BRL) (hereinafter simply referred to as "SHRPMI medium") so as to have a concentration of 2 × 10⁶ cells/ml. Thereafter, the suspension was spread on a 24-well cell culture plate (manufactured by Falcon) in a volume of 1 ml/well, and the cells were incubated in a wet-type incubator at 37°C and 5% CO₂ for 1.5 hours. After 1.5 hours, the non-adherent cells were removed by suction, and each well was washed with RPMI 1640 medium, and 1 ml of the 5HRPMI medium was added to the wells, to give monocytes.

### (2) TNFα ELISA

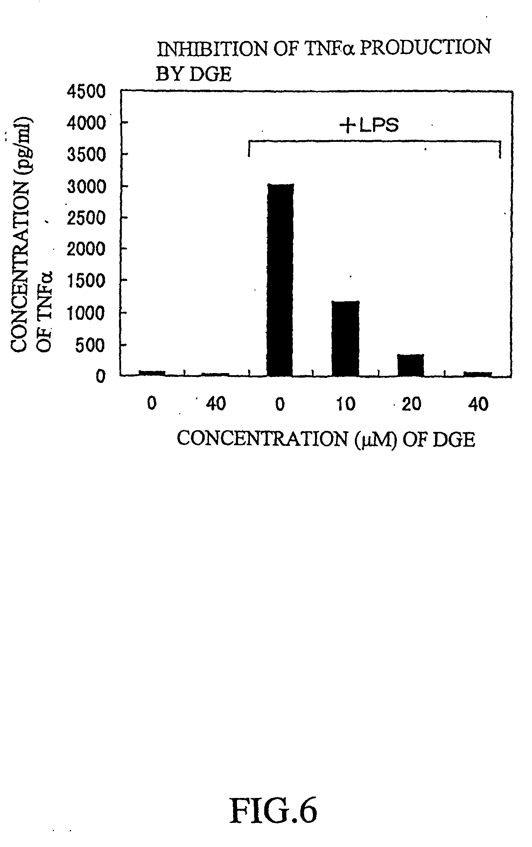

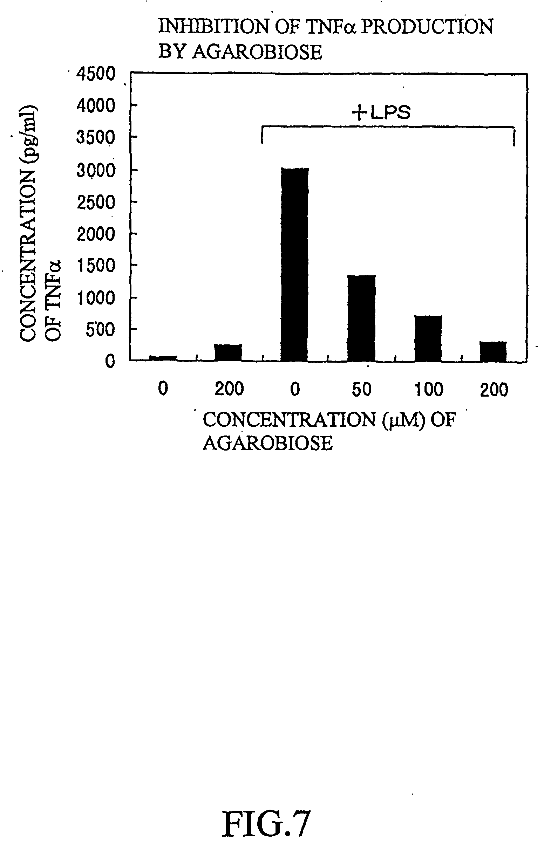

To the monocytes obtained above was added in each well 10 µl of a 1 mM, 2 mM or 4 mM aqueous DGE solution (each having a final concentration of 10 µM, 20 µM or 40 µM), or a 5 mM, 10 mM or 20 mM aqueous agarobiose solution (each having a final concentration of 50 µM, 100 µM or 200 µM), or 10 µl of water as a control (i.e. each of concentrations of DGE and agarobiose being 0 µM), and the cells were incubated in a wet-type incubator at 37°C and 5% CO₂ for 5 hours. Thereafter, with addition of 5 µl of an aqueous 200 ng/ml LPS solution to each well, the cells were cultured for additional 4 hours, and thereafter, the culture supernatant was recovered. The content of TNFα in the culture supernatant was assayed by enzyme immuno-sandwich assay (ELISA: Human TNFα ELISA Kit, manufactured by Endogen, EH2-TNFA). Here, the group with addition of water alone, the aqueous DGE solution alone (final concentration of 40 µM), or aqueous agarobiose solution alone (final concentration of 200 µM) to the cells was set as a negative control. The group with addition of water and the aqueous LPS solution to the cells was set as a positive control. In addition, all of the assays were carried out twice. As a result, there was found inhibition of LPS-stimulated TNFα production DGE concentration- or agarobiose concentration-dependently in the group with addition of DGE or agarobiose. The results are shown in Figures 6 and 7. Concretely, Figure 6 is a graph showing a concentration of TNFα in the culture supernatant when the cells were cultured under each of the conditions of with or without addition of DGE, and Figure 7 is a graph showing a concentration of TNFα in the culture supernatant when the cells were cultured under each of the conditions of with or without addition of agarobiose. In Figures 6 and 7, the axis of abscissas is culture conditions, and the axis of ordinates is the concentration of TNFα (pg/ml).

### Example 6

### (1) Synthesis of Primers

Oligonucleotide primers for reaction of PCR were designed based on mRNA nucleotide sequence of each gene listed in the following tables, and synthesized in the same manner as above. In Tables 4 and 5, each gene evaluated in this Example, GenBank Accession NO, the name of oligonucleotide primers and SEQ ID NOs of the primers in Sequence Listing were listed. As an internal standard, oligonucleotide primer AC3 of SEQ ID NO: 61 of Sequence Listing was synthesized based on mRNA nucleotide sequence of human β-actin [GenBank Accession NO: M10277].

**Table 4**

| Gene Name (Abbreviation) | GenBank Accession No. | Name of Primers (SEQ ID NOs) |
|---|---|---|
| - Cytokine and Cytokine Receptor | | |
| interleukin-6 (IL-6) | M14584 | IL6-F (SEQ ID NO: 9) |
| | | IL6-R (SEQ ID NO: 10) |
| | | |
| interleukin-1α (IL-1α) | X02851 | IL1α-F (SEQ ID NO: 11) |
| | | IL1α-R (SEQ ID NO: 12) |
| | | |
| interleukin-1β (IL-1β) | K02770 | IL1β-F (SEQ ID NO: 13) |
| | | IL1β-R (SEQ ID NO: 14) |
| | | |
| tumor necrosis factor α (TNFα) | M10988 | TNF-F (SEQ ID NO: 15) |
| | | TNF-R (SEQ ID NO: 16) |
| | | |
| interleukin-7 receptor (IL-7r) | M29696 | IL7r-F (SEQ ID NO: 17) |
| | | IL7r-R (SEQ ID NO: 18) |

| - CC Chemokine | | |
|---|---|---|
| macrophage inflammatory protein-1-β (MIP1β) | J04130 | MIP1β-F (SEQ ID NO: 19) |
| | | MIP1β-R (SEQ ID NO: 20) |
| | | |
| liver and activation-regulated chemokine (LARC) | D86955 | LARC-F (SEQ ID NO: 21) |
| | | LARC-R (SEQ ID NO: 22) |
| | | |
| macrophage-derived chemokine (MDC) | U83171 | MDC-F (SEQ ID NO: 23) |
| | | MDC-R (SEQ ID NO: 24) |

| - CXC Chemokine | | |
|---|---|---|
| macrophage inflammatory protein-2-β (MIP2β) | M36821 | MIP2β-F (SEQ ID NO: 25) |
| | | MIP2β-R (SEQ ID NO: 26) |
| | | |
| macrophage inflammatory protein-2-α (MIP2α) | M36820 | MIP2α-F (SEQ ID NO: 27) |
| | | MIP2α-R (SEQ ID NO: 28) |
| | | |
| growth regulated protein-1 (Gro1) | J03561 | Gro1-F (SEQ ID NO: 29) |
| | | Gro1-R (SEQ ID NO: 30) |

**Table 5**

| Gene Name (Abbreviation) | GenBank Accession No. | Name of Primers (SEQ ID NOs) |
|---|---|---|
| - Others | | |
| matrix metalloproteinase-9 (MMP-9) | J05070 | MMP9-F (SEQ ID NO: 31) |
| | | MMP9-R (SEQ ID NO: 32) |
| | | |
| migration inhibitory factor-related protein-8 (MRP8) | X06234 | MRP8-F (SEQ ID NO: 33) |
| | | MRP8-R (SEQ ID NO: 34) |
| | | |
| lyzozyme (Lyz) | M21119 | Lyz-F (SEQ ID NO: 35) |
| | | Lyz-R (SEQ ID NO: 36) |
| | | |
| GABA(A) receptor-associated protein (GABArp) | NM007278 | GABArp-F (SEQ ID NO: 37) |
| | | GABArp-R (SEQ ID NO: 38) |
| | | |
| interferon-induced 17-kDa/ 15-kDa protein (IFNp15/p17) | M13755 | IFNp15/p17-F (SEQ ID NO: 39) |
| | | IFNp15/p17-R (SEQ ID NO: 40) |
| | | |
| interferon-inducible protein p78 (IFNp78) | M33882 | IFNp78-F (SEQ ID NO: 41) |
| | | IFNp78-R (SEQ ID NO: 42) |
| | | |
| SCO(cytochrome oxidase deficient, yeast) homolog-2 (SCO2) | AL021683 | SCO2-F (SEQ ID NO: 43) |
| | | SCO2-R (SEQ ID NO: 44) |
| | | |
| transketolase (TK) | L12711 | TK-F (SEQ ID NO: 45) |
| | | TK-R (SEQ ID NO: 46) |
| | | |
| adenosine A2a receptor (ADORA2A) | S46950 | ADORA2A-F (SEQ ID NO: 47) |
| | | ADORA2A-R (SEQ ID NO: 48) |
| | | |
| CD37 antigen (CD37) | X14046 | CD37-F (SEQ ID NO: 49) |
| | | CD37-R (SEQ ID NO: 50) |
| | | |
| properdin P factor, complement (PFC) | M83652 | PFC-F (SEQ ID NO: 51) |
| | | PFC-R (SEQ ID NO: 52) |
| | | |
| regulator of G-protein signaling-2 (RGS2) | L13463 | RGS2-F (SEQ ID NO: 53) |
| | | RGS2-R (SEQ ID NO: 54) |
| | | |
| Nef-associated factor-1 (NAF1) | AJ011896 | NAF1-F (SEQ ID NO: 55) |
| | | NAF1-R (SEQ ID NO: 56) |
| | | |
| myeloid leukemia cell differentiation protein-1 (MCL1) | L08246 | MCL1-F (SEQ ID NO: 57) |
| | | MCL1-R (SEQ ID NO: 58) |
| | | |
| signal peptidase complex (18kDa) (SPC18) | AF061737 | SPC18-F (SEQ ID NO: 59) |
| | | SPC18-R (SEQ ID NO: 60) |

### (2) DNA Fragment Amplification by PCR Method with cDNA as Template

A reaction for PCR was carried out in the same manner as in item (6) of Example 4, using each of the oligonucleotide primers listed above and the oligonucleotide primer AC1 or AC2 of human β-actin synthesized in item (5) of Example 4, or the above-mentioned AC3, and a cDNA solution synthesized in the same manner as in item (4) of Example 4. The number of cycles for PCR was 30 cycles or 35 cycles as listed in the following tables. The reacted sample was frozen at -20°C and stored until the sample was analyzed.

Five microliters of each PCR mixture was analyzed by 3.0% agarose gel electrophoresis. The results are shown in Tables 6 and 7. The expression level of each gene in the tables (evaluated by an extent of amplification of mRNA-derived cDNA of each gene by PCR) was indicated in five stages in the ascending order of -, +/-, +, ++ and +++. In addition, in the tables, Con. is control (the group with addition of water), DGE is the group with addition of DGE, LPS is the group with addition of LPS, L + D is the group with addition of DGE and LPS, TPA is the group with addition of TPA, T + D is the group with addition of DGE and TPA.

**Table 6**

| Gene | Con. | DGE | LPS | L+D | TPA | T+D | Number of Cycles |
|---|---|---|---|---|---|---|---|
| - Cytokine and Cytokine Receptor | | | | | | | |
| EL-6 | - | - | +++ | - | + | - | 30 |
| IL-1α | +/- | - | +++ | +/- | + | - | 30 |
| IL-1β | + | + | +++ | + | ++ | + | 30 |
| TNFα | +/- | - | ++ | +/- | + | +/- | 30 |
| IL-7r | +/- | - | ++ | - | - | - | 30 |

| - CC Chemokine | | | | | | | |
|---|---|---|---|---|---|---|---|
| MIP1β | + | +/- | +++ | +/- | ++ | +/- | 30 |
| LARC | +/- | +/- | +++ | + | ++ | + | 30 |
| MDC | - | - | + | - | - | - | 30 |

| - CXC Chemokine | | | | | | | |
|---|---|---|---|---|---|---|---|
| MIP2β | + | + | ++ | + | ++ | + | 30 |
| MIP2α | +/- | +/- | + | +/- | + | +/- | 35 |
| Gro1 | + | + | +++ | + | ++ | + | 30 |

**Table 7**

| Gene | Con. | DGE | LPS | L+D | TPA | T+D | Number of Cycles |
|---|---|---|---|---|---|---|---|
| - Others | | | | | | | |
| MMP-9 | - | - | - | - | + | - | 30 |
| MRP8 | + | + | +/- | + | +/- | + | 30 |
| Lyz | + | + | +/- | + | +/- | + | 30 |
| GABArp | + | + | +/- | + | + | + | 30 |
| IFNp15/p17 | + | + | +++ | +/- | + | +/- | 30 |
| IFNp78 | + | +/- | ++ | - | + | +/- | 30 |
| SCO2 | + | + | - | + | + | + | 30 |
| TK | +/- | +/- | +/- | + | - | + | 30 |
| ADORA2A | + | +/- | +++ | - | + | - | 35 |
| CD37 | +/- | +/- | - | + | +/- | +/- | 35 |
| PFC | + | + | - | + | + | + | 35 |
| RGS2 | +/- | +/- | - | +/- | +/- | +/- | 35 |
| NAF1 | + | - | ++ | +/- | +/- | - | 30 |
| MCL1 | + | + | +/- | + | + | + | 30 |
| SPC18 | + | + | +/- | + | + | + | 30 |

It can be seen from the above results that when stress-stimulation is loaded to cells by LPS or TPA, DGE acts to lower the expression level for those genes of which expression levels are increased by the stimulation, while DGE acts to increase the expression level for those genes of which expression level is decreased by the stimulation. In other words, it is found that DGE acts so that the homeostasis of the living body is recovered.

### SEQUENCE LISTING FREE TEXT

SEQ ID NOs: 1 and 2 are sequences for primers designed based on a nucleotide sequence of human COX2 mRNA.

SEQ ID NOs: 3 and 4 are sequences for primers designed based on a nucleotide sequence of human MIP1α mRNA.

SEQ ID NOs: 5 and 6 are sequences for primers designed based on a nucleotide sequence of human RANTES mRNA.

SEQ ID NOs: 7 and 8 are sequences for primers designed based on a nucleotide sequence of human β-actin mRNA.

SEQ ID NOs: 9 and 10 are sequences for primers designed based on a nucleotide sequence of human IL-6 mRNA.

SEQ ID NOs: 11 and 12 are sequences for primers designed based on a nucleotide sequence of human IL-1α mRNA.

SEQ ID NOs: 13 and 14 are sequences for primers designed based on a nucleotide sequence of human IL-1β mRNA.

SEQ ID NOs: 15 and 16 are sequences for primers designed based on a nucleotide sequence of human TNFα mRNA.

SEQ ID NOs: 17 and 18 are sequences for primers designed based on a nucleotide sequence of human IL-7r mRNA.

SEQ ID NOs: 19 and 20 are sequences for primers designed based on a nucleotide sequence of human MIP1β mRNA.

SEQ ID NOs: 21 and 22 are sequences for primers designed based on a nucleotide sequence of human LARC mRNA.

SEQ ID NOs: 23 and 24 are sequences for primers designed based on a nucleotide sequence of human MDC mRNA.

SEQ ID NOs: 25 and 26 are sequences for primers designed based on a nucleotide sequence of human MIP2β mRNA.

SEQ ID NOs: 27 and 28 are sequences for primers designed based on a nucleotide sequence of human MIP2α mRNA.

SEQ ID NOs: 29 and 30 are sequences for primers designed based on a nucleotide sequence of human Gro1 mRNA.

SEQ ID NOs: 31 and 32 are sequences for primers designed based on a nucleotide sequence of human MMP-9 mRNA.

SEQ ID NOs: 33 and 34 are sequences for primers designed based on a nucleotide sequence of human MRP8 mRNA.

SEQ ID NOs: 35 and 36 are sequences for primers designed based on a nucleotide sequence of human Lyz mRNA.

SEQ ID NOs: 37 and 38 are sequences for primers designed based on a nucleotide sequence of human GABArp mRNA.

SEQ ID NOs: 39 and 40 are sequences for primers designed based on a nucleotide sequence of human IFN p15/p17 mRNA.

SEQ ID NOs: 41 and 42 are sequences for primers designed based on a nucleotide sequence of human IFN p78 mRNA.

SEQ ID NOs: 43 and 44 are sequences for primers designed based on a nucleotide sequence of human SCO2 mRNA.

SEQ ID NOs: 45 and 46 are sequences for primers designed based on a nucleotide sequence of human TK mRNA.

SEQ ID NOs: 47 and 48 are sequences for primers designed based on a nucleotide sequence of human ADORA2A mRNA.

SEQ ID NOs: 49 and 50 are sequences for primers designed based on a nucleotide sequence of human CD37 mRNA.

SEQ ID NOs: 51 and 52 are sequences for primers designed based on a nucleotide sequence of human PFC mRNA.

SEQ ID NOs: 53 and 54 are sequences for primers designed based on a nucleotide sequence of human RGS2 mRNA.

SEQ ID NOs: 55 and 56 are sequences for primers designed based on a nucleotide sequence of human NAF1 mRNA.

SEQ ID NOs: 57 and 58 are sequences for primers designed based on a nucleotide sequence of human MCL1 mRNA.

SEQ ID NOs: 59 and 60 are sequences for primers designed based on a nucleotide sequence of human SPC18 mRNA.

SEQ ID NO: 61 is a sequence for primer designed based on a nucleotide sequence of human β-actin mRNA.

### INDUSTRIAL APPLICABILITY

As described above, according to the present invention, there is provided a suppressor for unbalance of gene expressions, which is useful for those necessary for sustaining homeostasis of a living body, for instance, regulation of interleukin production or regulation of production of *in vivo* enzymes, and also is provided a therapeutic agent or prophylactic agent for a disease that requires modulating unbalance of gene expressions of various biological factors, comprising the suppressor, whereby the disease caused by abnormality of the production of the factor can be treated or prevented.

In addition, according to the present invention, there is provided a method for modulating unbalance of gene expressions of various biological factors. The method comprises administering the above-mentioned suppressor or the like, thereby inducing or inhibiting production of the desired factor, whereby the homeostasis in a living body can be recovered. In this method, the above-mentioned suppressor or the like is suitably used as an inducer or inhibitor for production of various factors. Further, as one embodiment of the present invention, there is provided a screening method for a therapeutic agent or prophylactic agent for a disease involved with a cytokine or *in vivo* enzyme.

## Claims

1. A suppressor for unbalance of gene expressions, **characterized in that** the suppressor comprises, as an effective ingredient, at least one compound selected from a compound represented by the following formula (I): 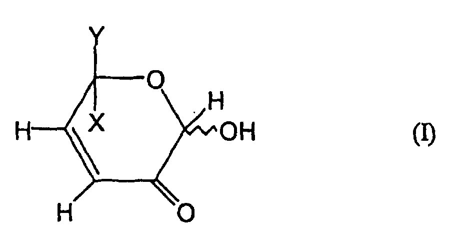 wherein X and Y are H or CH₂OH, with proviso that when X is CH₂OH, Y is H, and when X is H, Y is CH₂OH;
a compound represented by the following formula (II): 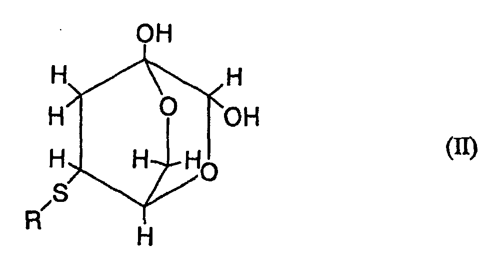 wherein R is a residue resulting from removal of one SH group from an SH-group containing compound;
and a salt thereof.

2. The suppressor for unbalance of gene expressions according to claim 1, wherein unbalance of gene expressions of one or more genes selected from the group consisting of:
interleukin-6 (GenBank Accession NO: M14584) gene,
interleukin-10 (GenBank Accession NO: M57627) gene,
hemeoxygenase-1 (GenBank Accession NO: NM 002133) gene,
prostaglandin G/H synthase-2 (GenBank Accession NO: AL033533) gene,
macrophage inflammatory protein-1-α (GenBank Accession NO: M23452) gene,
RANTES (GenBank Accession NO: NM 002985) gene,
interleukin-1α (GenBank Accession NO: X02851) gene,
interleukin-1β (GenBank Accession NO: K02770) gene,
tumor necrosis factor α (GenBank Accession NO: M10988) gene,
interleukin-7 receptor (GenBank Accession NO: M29696) gene,
macrophage inflammatory protein-1-β (GenBank Accession NO: J04130) gene,
liver and activation-regulated chemokine (GenBank Accession NO: D86955) gene,
macrophage-derived chemokine (GenBank Accession NO: U83171) gene,
macrophage inflammatory protein-2-β (GenBank Accession NO: M36821) gene,
macrophage inflammatory protein-2-α (GenBank Accession NO: M36820) gene,
growth regulated protein-1 (GenBank Accession NO: J03561) gene,
matrix metalloproteinase-9 (GenBank Accession NO: J05070) gene,
migration inhibitory factor-related protein-8 (GenBank Accession NO: X06234) gene,
lyzozyme (GenBank Accession NO: M21119) gene,
GABA(A) receptor-associated protein (GenBank Accession NO: NM 007278) gene,
interferon-induced 17-kDa/15-kDa protein (GenBank Accession NO: M13755) gene,
interferon-inducible protein p78 (GenBank Accession NO: M33882) gene,
SCO(cytochrome oxidase deficient, yeast) homolog-2 (GenBank Accession NO: AL021683) gene,
transketolase (GenBank Accession NO: L12711) gene,
adenosine A2a receptor (GenBank Accession NO: S46950) gene,
CD37 antigen (GenBank Accession NO: X14046) gene,
properdin P factor, complement (GenBank Accession NO: M83652) gene,
regulator of G-protein signaling-2 (GenBank Accession NO: L13463) gene,
Nef-associated factor-1 (GenBank Accession NO: AJ011896) gene,
myeloid leukemia cell differentiation protein-1 (GenBank Accession NO: L08246) gene, and
signal peptidase complex (18 kDa) (GenBank Accession NO: AF061737) gene is modulated.

3. A therapeutic agent or prophylactic agent for a disease that requires modulating unbalance of gene expressions of one or more genes selected from a group of genes listed in claim 2, comprising, as an effective ingredient, at least one compound selected from a compound represented by the following formula (I): 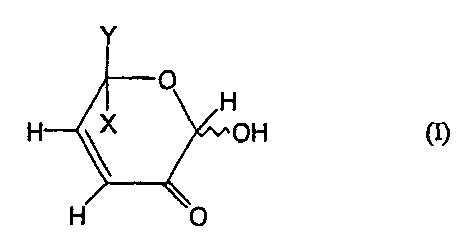 wherein X and Y are H or CH₂OH, with proviso that when X is CH₂OH, Y is H, and when X is H, Y is CH₂OH;
a compound represented by the following formula (II): 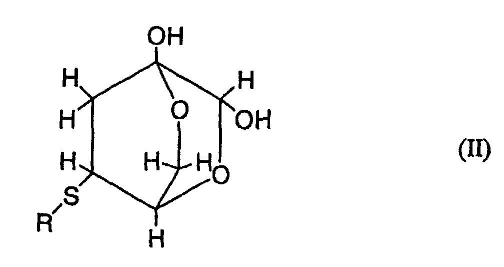 wherein R is a residue resulting from removal of one SH group from an SH-group containing compound;
and a salt thereof.

4. The therapeutic agent or prophylactic agent according to claim 3, wherein the disease is an inflammatory disease.

5. The therapeutic agent or prophylactic agent according to claim 3 or 4, wherein the therapeutic agent or prophylactic agent is an inhibitor for interleukin-6 production, an inhibitor for interleukin-10 production, an inducer for hemeoxygenase production, an inhibitor for prostaglandin G/H synthase-2 production, an inhibitor for macrophage inflammatory protein-1-α production, an inhibitor for RANTES production, or an inhibitor for tumor necrosis factor α production.

6. A method for modulating unbalance of gene expressions, **characterized by** administering to a mammal at least one compound selected from a compound represented by the following formula (I): 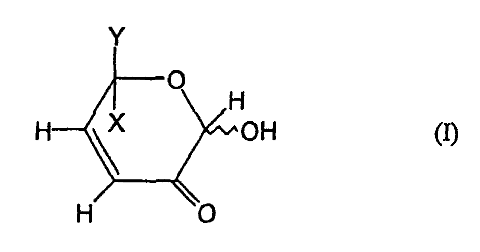 wherein X and Y are H or CH₂OH, with proviso that when X is CH₂OH, Y is H, and when X is H, Y is CH₂OH;
a compound represented by the following formula (II): 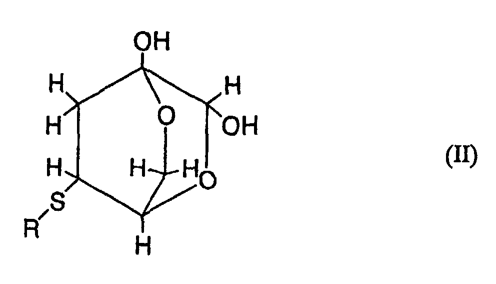 wherein R is a residue resulting from removal of one SH group from an SH-group containing compound;
and a salt thereof.

7. The method for modulating unbalance of gene expressions according to claim 6, wherein unbalance of gene expressions of one or more genes selected from a group of genes listed in claim 2 is modulated.
